# EUROPÄISCHE PATENTSCHRIFT

(11) **EP 0 619 290 B1**
(45) Veröffentlichungstag und Bekanntmachung des Hinweises auf die Patenterteilung: **09.10.1996**
(21) Anmeldenummer: 94810183.7
(22) Anmeldetag: 23.03.1994
(51) Int. Cl.: C07C 59/84, C07C 59/88, C07C 51/41, C09D 5/08

(54) **Erdalkalimetallsalze, Uebergangsmetallsalze und Uebergangsmetallkomplexe von Ketocarbonsäuren als Korrosionsinhibitoren**
Salts of alkaline earth metals and transition metals as well as complexes of transition metals of ketocarboxylic acids and their use as corrosion inhibitors
Sels de métaux alcalino-terreux et de transition ainsi que les complexes de métaux de transition d'acides cétocarboxyliques et leur emploi en tant qu'inhibiteurs de corrosion

(30) Priorität: 07.04.1993 CH 1059/93
(43) Veröffentlichungstag der Anmeldung: 12.10.1994
(73) Patentinhaber: CIBA-GEIGY AG, 4002 Basel (CH)
(72) Erfinder: Armstrong, William Paul, Dr., Belfast BT148AH (GB); Braig, Adalbert, Dr., D-79589 Binzen (DE); Frey, Markus, Dr., CH-1723 Marly (CH); Kramer, Andreas, Dr., CH-3186 Düdingen (CH)

(56) Entgegenhaltungen:
- EP-A- 0 144 663
- EP-A- 0 300 325
- EP-A- 0 412 933
- EP-A- 0 496 555
- DE-A- 2 021 445
- US-A- 3 382 081

## Beschreibung

Die vorliegende Erfindung betrifft neue Erdalkalimetallsalze, Uebergangsmetallsalze und Uebergangsmetallkomplexe von Ketocarbonsäuren, Ueberzugszusammensetzungen enthaltend ein organisches filmbildendes Bindemittel, bevorzugt ein Anstrichmittel und die neuen Korrosionsinhibitoren, sowie die Verwendung derselben in Ueberzugszusammensetzungen zum Schutz von metallischen Oberflächen.

Die Verwendung von Alkali-, Ammonium- und Amin-Salzen von Ketocarbonsäuren als Korrosionsinhibitoren in wässrigen Systemen ist bekannt und beispielsweise in US-A-4 909 987, EP-A-412 933 oder EP-A-496 555 beschrieben.

Die Verwendung der Calciumsalze der 3-(3-Chlor-4-isopropyl-benzoyl)propionsäure und der 3-(3-Chlor-4-cyclohexyl-benzoyl)propionsäure zur Herstellung von analgetischen und/oder entzündungshemmenden Arzneimitteln ist in DE-A-2 021 445 offenbart.

Die Verwendung des Calcium-, Magnesium- und Zinksalzes der 4-Benzoylbuttersäure zur Förderung des Wachstums von Tieren ist bekannt und beispielsweise in EP-A-0 300 325 beschrieben.

Es wurde nun gefunden, dass Erdalkalimetallsalze, Uebergangsmetallsalze und Uebergangsmetallkomplexe von Ketocarbonsäuren sich besonders gut als Korrosionsinhibitoren in Ueberzugszusammensetzungen zum Schutz von metallischen Oberflächen eignen.

Die vorliegende Erfindung betrifft daher Erdalkalimetallsalze, Uebergangsmetallsalze und Uebergangsmetallkomplexe von Verbindungen der Formel I 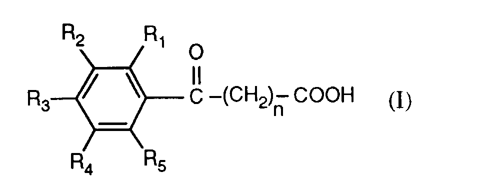 worin R₁, R₂, R₃, R₄ und R₅ unabhängig voneinander Wasserstoff, Halogen, Nitro, Cyano, CF₃, C₁-C₁₅-Alkyl, C₅-C₁₂-Cycloalkyl, C₂-C₁₅-Alkenyl, C₁-C₁₂-Halogenalkyl, C₁-C₁₂-Alkoxy, C₁-C₁₂-Alkylthio, unsubstituiertes oder durch C₁-C₄-Alkyl substituiertes C₆-C₁₀-Aryl; unsubstituiertes oder durch C₁-C₄-Alkyl substituiertes C₆-C₁₀-Aryloxy; unsubstituiertes oder am Arylrest durch 1 bis 3 C₁-C₄-Alkyl substituiertes C₇-C₁₂-Arylalkyl; -CO₂R₆, -COR₆ oder 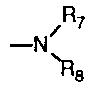 darstellen, wobei mindestens einer der Reste R₁ bis R₅ Wasserstoff, Halogen oder C₁-C₁₅-Alkyl bedeutet, ferner die Reste R₁ und R₂, R₂ und R₃, R₃ und R₄ oder R₄ und R₅ zusammen mit den Kohlenstoffatomen, an die sie gebunden sind, einen Benzo- oder Cyclohexenyl-Ring bilden,
R₆ C₁-C₂₀-Alkyl, durch Sauerstoff, Schwefel oder 〉N-R₉ unterbrochenes C₂-C₂₀-Alkyl; unsubstituiertes oder am Arylrest durch 1 bis 3 C₁-C₄-Alkyl substituiertes C₇-C₁₂-Arylalkyl bedeutet,
R₇ und R₈ unabhängig voneinander Wasserstoff, C₁-C₂₄-Alkyl oder durch Sauerstoff, Schwefel oder 〉N-R₉ unterbrochenes C₂-C₂₄-Alkyl darstellen,
R₉ Wasserstoff oder C₁-C₈-Alkyl bedeutet, und
n eine ganze Zahl aus dem Bereich von 1 bis 10 darstellt; mit der Bedingung, dass das Calciumsalz der 3-(3-Chlor-4-isopropyl-benzoyl)propionsäure, das Calciumsalz der 3-(3-Chlor-4-cyclohexyl-benzoyl)propionsäure und das Calcium-, Magnesium- und Zinksalz der 4-Benzoylbuttersäure ausgeschlossen sind.

Halogen bedeutet beispielsweise Fluor, Chlor, Brom oder Iod. Bevorzugt sind Fluor, Chlor oder Brom, insbesondere Chlor oder Brom.

Alkyl mit bis zu 24 Kohlenstoffatomen bedeutet einen verzweigten oder unverzweigten Rest wie beispielsweise Methyl, Ethyl, Propyl, Isopropyl, n-Butyl, sec-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl oder Docosyl.

C₅-C₁₂-Cycloalkyl bedeutet beispielsweise Cyclopentyl, Cyclohexyl, Cycloheptyl, Cyclooctyl, Cyclodecyl oder Cyclodocecyl. Bevorzugt ist Cyclohexyl.

Alkenyl mit 2 bis 15 Kohlenstoffatomen bedeutet einen verzweigten oder unverzweigten Rest wie beispielsweise Vinyl, 2-Propenyl (Allyl), 2-Butenyl, 3-Butenyl, Isobutenyl, n-2,4-Pentadienyl, 3-Methyl-2-butenyl, n-2-Octenyl, n-2-Dodecenyl oder iso-Dodecenyl.

Halogenalkyl mit bis zu 12 Kohlenstoffatomen bedeutet einen verzweigten oder unverzweigten Rest wie beispielsweise Chlormethyl, Bromethyl, Fluorpropyl, Chlorpentyl, Chlorhexyl, Chloroctyl, Chlordecyl oder Chlordodecyl.

Alkoxy mit bis zu 12 Kohlenstoffatomen bedeutet einen verzweigten oder unverzweigten Rest wie beispielsweise Methoxy, Ethoxy, Propoxy, Isopropoxy, n-Butoxy, Isobutoxy, Pentoxy, Isopentoxy, Hexoxy, Heptoxy, Octoxy oder Decyloxy.

Alkylthio mit bis zu 12 Kohlenstoffatomen bedeutet einen verzweigten oder unverzweigten Rest wie beispielsweise Methylthio, Ethylthio, Propylthio, Isopropylthio, n-Butylthio, Isobutylthio, Pentylthio, Isopentylthio, Hexylthio, Heptylthio, Octylthio, Decylthio oder Dodecylthio.

Unsubstituiertes oder durch C₁-C₄-Alkyl substituiertes C₆-C₁₀-Aryl, das vorzugsweise 1 bis 3, insbesondere 1 oder 2 Alkylgruppen trägt, bedeutet beispielsweise Phenyl, Naphthyl, o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 4-tert-Butylphenyl, 2-Ethylphenyl, 2,6-Diethylphenyl, 2-Methylnaphthyl, 1-MethyInaphthyl, 4-Methylnaphthyl, 2-Ethylnaphthyl oder 2,6-Diethylnaphthyl.

Unsubstituiertes oder durch C₁-C₄-Alkyl substituiertes C₆-C₁₀-Aryloxy, das vorzugsweise 1 bis 3, insbesondere 1 oder 2 Alkylgruppen trägt, bedeutet beispielsweise Phenoxy, Naphthoxy, o-, m- oder p-Methylphenoxy, 2,3-Dimethylphenoxy, 2,4-Dimethylphenoxy, 2,5-Dimethylphenoxy, 2,6-Dimethylphenoxy, 3,4-Dimethylphenoxy, 3,5-Dimethylphenoxy, 2-Methyl-6-ethylphenoxy, 4-tert-Butylphenoxy, 2-Ethylphenoxy, 2,6-Diethylphenoxy, 2-Methylnaphthoxy, 1-Methylnaphthoxy, 4-Methylnaphthoxy, 2-Ethylnaphthoxy oder 2,6-Diethylnaphthoxy.

Unsubstituiertes oder am Arylrest durch 1 bis 3 C₁-C₄-Alkyl substituiertes C₇-C₁₂-Arylalkyl bedeutet beispielsweise Phenyl-C₁-C₆-alkyl oder Naphthyl-C₁-C₂-alkyl wie etwa Benzyl, 4-Methylbenzyl, 4-tert-Butylbenzyl, 2,4-Dimethylbenzyl, α-Methylbenzyl, α,α-Dimethylbenzyl, 2-Phenylethyl, 2-Naphthylmethyl, 1-Naphthylmethyl, 1-Naphthylethyl oder 2-Naphthylethyl. Bevorzugt ist Benzyl.

Durch Sauerstoff, Schwefel oder 〉N-R₉ unterbrochenes Alkyl mit 2 bis zu 24 Kohlenstoffatomen bedeutet beispielsweise CH₃-O-CH₂-, CH₃-S-CH₂-, CH₃-NH-CH₂-, CH₃-N(CH₃)-CH₂-, CH₃-O-CH₂CH₂-O-CH₂-, CH₃-(O-CH₂CH₂-)₂O-CH₂-, CH₃-(O-CH₂CH₂-)₃O-CH₂- oder CH₃-(O-CH₂CH₂-)₄O-CH₂-.

Die Erdalkalimetalle bedeuten beispielsweise Magnesium, Calcium, Strontium oder Barium. Bevorzugt ist Calcium.

Die Uebergangsmetalle bedeuten die Elemente 21 bis 30, 39 bis 48 und 57 bis 80 des Periodensystems. Bevorzugt sind Titan, Mangan, Eisen, Kobalt, Zink, Yttrium, Zirkonium, Lanthan oder Cer. Speziell bevorzugt ist Zirkonium.

Bevorzugt sind Verbindungen der Formel I, worin
R₁, R₂, R₃, R₄ und R₅ unabhängig voneinander Wasserstoff, Halogen, Nitro, Cyano, CF₃, C₁-C₁₅-Alkyl, C₅-C₁₂-Cycloalkyl, C₂-C₁₅-Alkenyl, C₁-C₁₂-Halogenalkyl, C₁-C₁₂-Alkoxy, C₁-C₁₂-Alkylthio, C₆-C₁₀-Aryl, C₆-C₁₀-Aryloxy, C₇-C₁₂-Arylalkyl, -CO₂R₆, -COR₆ oder 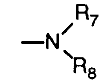 darstellen, wobei mindestens einer der Reste R₁ bis R₅ Wasserstoff, Halogen oder C₁-C₁₅-Alkyl bedeutet, ferner die Reste R₁ und R₂, R₂ und R₃, R₃ und R₄ oder R₄ und R₅ zusammen mit den Kohlenstoffatomen, an die sie gebunden sind, einen Benzo- oder Cyclohexenyl-Ring bilden, und
R₆ C₁-C₂₀-Alkyl, durch Sauerstoff, Schwefel oder 〉N-R₉ unterbrochenes C₂-C₂₀-Alkyl;
oder C₇-C₁₂-Arylalkyl bedeutet.

Ebenso bevorzugt sind Verbindungen der Formel I, worin mindestens zwei, insbesondere 3 der Reste R₁ bis R₅ Wasserstoff sind.

Bevorzugt sind auch Verbindungen der Formel I, worin die Metalle Calcium, Titan, Mangan, Eisen, Kobalt, Zink, Yttrium, Zirkonium, Lanthan oder Cer darstellen.

Ebenfalls bevorzugt sind Verbindungen der Formel I, worin das Metall Zirkonium ist.

n ist in Formel I bevorzugt 1 bis 5, insbesondere 2.

Besonders bevorzugt sind Verbindungen der Formel I, worin R₁ Wasserstoff bedeutet, R₂, R₃, R₄ und R₅ unabhängig voneinander Wasserstoff, Chlor, Brom, Nitro, Cyano, CF₃, C₁-C₈-Alkyl, C₅-C₇-Cycloalkyl, C₂-C₈-Alkenyl, C₁-C₈-Alkoxy, C₁-C₈-Alkylthio, Phenyl, Phenyloxy, Benzyl, -CO₂R₆, -COR₆ oder 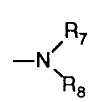 darstellen,
R₆ C₁-C₁₂-Alkyl, durch Sauerstoff unterbrochenes C₂-C₁₂-Alkyl; oder Benzyl bedeutet, R₇ und R₈ unabhängig voneinander Wasserstoff, C₁-C₈-Alkyl oder durch Sauerstoff unterbrochenes C₂-C₁₂-Alkyl darstellen, und
n eine ganze Zahl aus dem Bereich von 1 bis 5 bedeutet.

Ebenfalls besonders bevorzugt sind Verbindungen der Formel I, worin R₁, R₂ und R₄ Wasserstoff bedeuten,
R₃ und R₅ unabhängig voneinander Wasserstoff, Chlor, Brom, CF₃, C₁-C₈-Alkyl, Cyclohexyl, C₁-C₈-Alkoxy, -CO₂R₆, -COR₆ oder 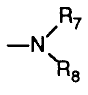 darstellen,
R₆ C₁-C₈-Alkyl bedeutet,
R₇ und R₈ unabhängig voneinander Wasserstoff oder C₁-C₈-Alkyl darstellen, und
n eine ganze Zahl aus dem Bereich von 2 bis 4 bedeutet.

Von besonderem Interesse sind Verbindungen der Formel I, worin R₁, R₂, R₄ und R₅ Wasserstoff bedeuten und n 2 ist.

Speziell von Interesse sind Verbindungen der Formel I, worin R₁, R₂, R₄ und R₅ Wasserstoff sind, R₃ Wasserstoff, C₁-C₄-Alkyl oder Chlor bedeutet, n 2 ist und die Metalle Calcium, Titan, Mangan, Eisen, Kobalt, Zink, Yttrium, Zirkonium, Lanthan oder Cer darstellen.

Ganz besonders bevorzugt sind die Erdalkalimetallsalze, Uebergangsmetallsalze und Uebergangsmetallkomplexe der 3-(4-Methylbenzoyl)propionsäure und der 3-(4-Chlorbenzoyl)propionsäure.

Die erfindungsgemässen Erdalkalimetallsalze, Uebergangsmetallsalze und Uebergangsmetallkomplexe von Verbindungen der Formel I können auf an sich bekannte Weise hergestellt werden.

Die Erfindung betrifft auch ein Verfahren zur Herstellung von Erdalkalimetallsalzen, Uebergangsmetallsalzen und Uebergangsmetallkomplexen von Verbindungen der Formel I, dadurch gekennzeichnet, dass eine Ketocarbonsäure der Formel I oder ein Alkalimetallsalz davon mit einer Erdalkalimetall- oder Uebergangsmetall-Verbindung umgesetzt wird.

Ein bevorzugtes Verfahren ist dadurch gekennzeichnet, dass als Erdalkalimetall- oder Uebergangsmetall-Verbindung ein Salz, eine metallorganische Verbindung oder eine anorganische Metallbase eingesetzt wird.

Bei der Herstellung von erfindungsgemässen Uebergangsmetallkomplexen, wie beispielsweise Zirkoniumkomplexen, ausgehend von Verbindungen der Formel I und Zirkoniumcarbonat wird die Reaktion in Wasser ohne Natriumhydroxid-Lösung bei erhöhter Temperatur, insbesondere Temperaturen von 50 bis 100°C, durchgeführt. Spezielle bevorzugt ist ein Temperaturbereich von 70 bis 95°C.

Die Reaktion kann aber auch in einem organischen Lösungsmittel, wie beispielsweise Toluol, bei leicht erhöhter Temperatur durchgeführt werden. Diese Methode ist speziell dann bevorzugt, wenn die Erdalkalimetall- und Uebergangsmetall-Verbindungen metallorganische Verbindungen sind. Bevorzugt wird die Reaktion in einem Temperaturbereich von 20 bis 70°C, insbesondere 40 bis 60°C durchgeführt.

Die Verbindungen der Formel I (freie Säuren) werden mit Erdalkali- und Uebergangsmetall-Verbindungen vorzugsweise in äquimolaren Mengen eingesetzt. Die erfindungsgemässen Erdalkalimetallsalze, Uebergangsmetallsalze und Uebergangsmetallkomplexe von Verbindungen der Formel I können z.B. mit der allgemeinen Formel II 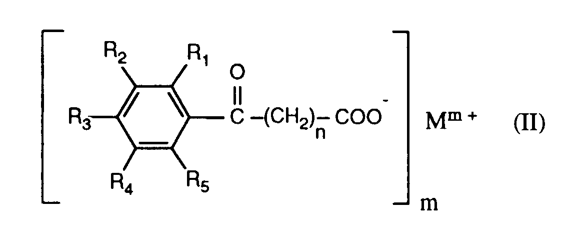 dargestellt werden, worin M ein Erdalkali- oder Uebergangs-Metall bedeutet und m 2, 3 oder 4 darstellt.

Im Falle von Uebergangsmetallsalzen und Uebergangsmetallkomplexen, insbesondere der Metalle Zirkonium und Titan, vorallem bei nicht äquimolarer Umsetzung, können die Endverbindungen auch von der Formel II abweichende Strukturen annehmen. Beispiele davon sind weiter unten für Zirkoniumkomplexe aufgeführt.

Im Falle von erfindungsgemässen Uebergangsmetallkomplexen, insbesondere von Zirkonium- und Titan-Komplexen, können die Verbindungen der Formel I (freie Säuren) bezüglich eingesetzter Zirkonium- und Titan-Verbindung im Ueberschuss, aequimolar, oder im Unterschuss verwendet werden. Das molare Verhältnis Ketocarbonsäure der Formel I zur Uebergangsmetall-Verbindung kann 20: 1 bis 1:10 betragen. Bevorzugt wird ein Verhältnis von 10:1 bis 1:3.

Die vorliegende Erfindung betrifft daher auch ein Verfahren zur Herstellung von erfindungsgemässen Verbindungen, dadurch gekennzeichnet, dass das molare Verhältnis der Ketocarbonsäure der Formel I zur Uebergangsmetall-Verbindung 20 : 1 bis 1 : 10 beträgt.

Ein speziell bevorzugtes Verfahren zur Herstellung von erfindungsgemässen Verbindungen ist dadurch gekennzeichnet, dass die Uebergangsmetall-Verbindung eine Zirkonium- oder Titan-Verbindung ist.

Die erfindungsgemässen Erdalkalimetallsalze, Uebergangsmetallsalze und Uebergangsmetallkomplexe von Verbindungen der Formel I können auch noch mit freier Säure (Formel I), Wasser oder mit anderen Anionen, wie Hydroxiden, die im Reaktionsmedium vorhanden sind, komplexiert sein. Im Falle von Metallacetaten oder Metallalkoxiden können enthalten sein.

Aufgrund der obigen Ausführungen kann der prozentuale Gewichts-Metallgehalt der erfindungsgemässen Verbindungen der Formel I unterschiedlich sein. So können die besonders bevorzugten erfindungsgemässen Zirkoniumkomplexe der 3-(4-Methylbenzoyl)propionsäure einen Zirkoniumgehalt von 5 bis 50 Gew.-%, vorzugsweise 10 bis 40 %, aufweisen.

Die Struktur der erfindungsgemässen Zirkoniumkomplexe ist nicht restlos aufgeklärt. Die Summenformeln der erfindungsgemässen Zirkoniumkomplexe der 3-(4-Methylbenzoyl)propionsäure (R = 3-(4-Methylbenzoyl)propionat) sind beispielsweise (vgl. Beispiele 2 bis 14): ZrO(OH)(O₂CR)·6,6 HO₂CR; ZrO(OH)(O₂CR)·2,9 HO₂CR;
ZrO(OH)(O₂CR)·0,3 HO₂CR; ZrO(OH)(O₂CR)·0,7 HO₂CR;
ZrO(OH)(O₂CR)_{0,8}(Acetat)_{0,2}; ZrO(OH)(O₂CR)_{0,5}(Acetat)_{0,5};
ZrO(OH)(O₂CR)· 0,3 HO₂CR; ZrO₂ · ZrO(OH)(O₂CR); Zr(OC₃H₇)₃(O₂CR); ZrO(OH)(O₂CR) · 0,48 HO₂CR; Zr(OC₃H₇)₂(O₂CR)₂; Zr(OC₄H₉)₃(O₂CR) oder Zr(OC₃H₇)₃(O₂CR).

Die vorliegende Erfindung betrifft daher auch Produkte, erhältlich durch Umsetzung einer Verbindung der Formel I oder einem Alkalimetallsalz davon mit einer Erdalkalimetall- oder Uebergangsmetall-Verbindung; mit der Bedingung, dass als Produkte das Calciumsalz der 3-(3-Chlor-4-isopropyl-benzoyl)propionsäure, das Calciumsalz der 3-(3-Chlor-4-cyclohexyl-benzoyl)propionsäure und das Calcium-, Magnesium- und Zinksalz der 4-Benzoylbuttersäure ausgeschlossen sind.

Besonders bevorzugt wird eine Verbindung der Formel I mit einer Base, z.B. mindestens einem Aequivalent einer wässrigen Alkalihydroxid-Lösung gelöst und anschliessend mit einer wässrigen Lösung einer Erdalkalimetall- oder Uebergangsmetall-Verbindung versetzt. Die erfindungsgemässen Verbindungen der Formel I werden filtriert oder aus dem Reaktionsmedium mit einem organischen Lösungsmittel, wie beispielsweise Essigsäureethylester oder Dichlormethan, extrahiert.

Die Verbindungen der Formel I (freie Säuren und nicht die erfindungsgemässen Salze und Komplexe) sind bekannt und viele sind im Handel erhältlich, oder können wie in Houben-Weyl, Methoden der Organischen Chemie, Band VIII, S. 381-382 (1952) und Band E5, S. 398-399 (1985) beschrieben, hergestellt werden. So liefert beispielsweise die Friedel-Crafts-Acylierung von substituierten Aromaten (Benzol- und Naphthalin-Derivate) mit cyclischen Anhydriden die Verbindungen der Formel I in ausgezeichneten Ausbeuten.

Die eingesetzten Erdalkalimetall- oder Uebergangsmetall-Verbindungen sind beispielsweise Salze, metallorganische Verbindungen oder anorganische Metallbasen oder Mischungen davon.

Beispiele für Salze sind Halogenide (insbesondere Chloride), Nitrate, Carbonate, Sulfate, wobei es sich auch um basiche Salze handeln kann, z.B. Lanthanchlorid, Yttriumchlorid, Eisenchlorid, Titantetrachlorid, Zinkchlorid, Calciumchlorid, Manganchlorid, Cobaltchlorid, Cerchlorid, Zinknitrat, Mangannitrat, Cobaltnitrat, Cernitrat, Eisennitrat, Calciumnitrat, Lanthannitrat, Yttriumnitrat, Zirkoniumsulfat, Eisensulfat, Mangansulfat, Cobaltsulfat, Cersulfat, Zirkoniumoxidchlorid, Titanoxidchlorid, Zirkoniumacetat, Zinkacetat, Manganacetat, Cobaltacetat, Lanthanacetat, Calciumacetat, Zirkoniumcarbonat, Zinkcarbonat, Mangancarbonat, Cobaltcarbonat und Calciumcarbonat.

Beispiele für metallorganische Verbindungen sind vorallem Alkoholate, z.B. Zirkonium-n-propoxid, Zirkonium-isopropoxid, Zirkonium n-butoxid, Titan-n-propoxid, Titan-isopropoxid, Titanethoxid und Titan-n-butoxid.

Beispiele for anorganische Metallbasen sind Hydroxide, Oxide und Amide, z.B. Calciumhydroxid, Calciumoxid und Calciumamid.

Als Alkalihydroxid-Lösung wird Kaliumhydroxid- oder Natriumhydroxid-Lösung, bevorzugt Natriumhydroxid-Lösung, verwendet.

Die Fällung der erfindungsgemässen Erdalkalimetallsalze und Uebergangsmetallkomplexe der Formel I erfolgt bevorzugt bei Raumtemperatur.

Die erfindungsgemässen Erdalkalimetallsalze, Uebergangsmetallsalze und Uebergangsmetallkomplexe von Verbindungen der Formel I eignen sich als Korrosionsinhibitoren in Ueberzugszusammensetzungen zum Schutz von metallischen Oberflächen aber auch zur Vorbehandlung metallischer Substrate. Als solche können sie allen flüssigen oder festen organischen Materialien zugesetzt werden.

Ein weiterer Gegenstand der Erfindung sind daher auch Ueberzugszusammensetzungen enthaltend a) ein organisches filmbildendes Bindemittel und b) mindestens ein Erdalkalimetallsalz, Uebergangsmetallsalz oder Uebergangsmetallkomplex von einer Verbindung der Formel I 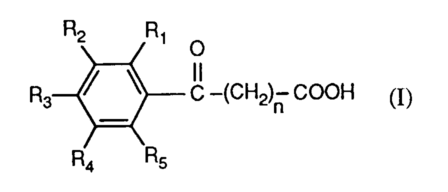 worin R₁, R₂, R₃, R₄ und R₅ unabhängig voneinander Wasserstoff, Halogen, Nitro, Cyano, CF₃, C₁-C₁₅-Alkyl, C₅-C₁₂-Cycloalkyl, C₂-C₁₅-Alkenyl, C₁-C₁₂-Halogenalkyl, C₁-C₁₂-Alkoxy, C₁-C₁₂-Alkylthio, unsubstituiertes oder durch C₁-C₄-Alkyl substituiertes C₆-C₁₀-Aryl; unsubstituiertes oder durch C₁-C₄-Alkyl substituiertes C₆-C₁₀-Aryloxy; unsubstituiertes oder am Arylrest durch 1 bis 3 C₁-C₄-Alkyl substituiertes C₇-C₁₂-Arylalkyl;
-CO₂R₆, -COR₆ oder 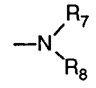 darstellen, wobei mindestens einer der Reste R₁ bis R₅ Wasserstoff, Halogen oder C₁-C₁₅-Alkyl bedeutet, ferner die Reste R₁ und R₂, R₂ und R₃, R₃ und R₄ oder R₄ und R₅ zusammen mit den Kohlenstoffatomen, an die sie gebunden sind, einen Benzo- oder Cyclohexenyl-Ring bilden,
R₆ C₁-C₂₀-Alkyl, durch Sauerstoff, Schwefel oder 〉N-R₉ unterbrochenes C₂-C₂₀-Alkyl; unsubstituiertes oder am Arylrest durch 1 bis 3 C₁-C₄-Alkyl substituiertes C₇-C₁₂-Arylalkyl bedeutet,
R₇ und R₈ unabhängig voneinander Wasserstoff, C₁-C₂₄-Alkyl oder durch Sauerstoff, Schwefel oder 〉N-R₉ unterbrochenes C₂-C₂₄-Alkyl darstellen,
R₉ Wasserstoff oder C₁-C₈-Alkyl bedeutet, und
n eine ganze Zahl aus dem Bereich von 1 bis 10 darstellt.

Bevorzugt ist die Ueberzugszusammensetzung ein Anstrichmittel. Speziell bevorzugt ist ein wässriges Anstrichmittel.

Anstrichmittel sind beispielsweise Lacke, Farben oder Firnisse. Diese enthalten stets ein organisches filmbildendes Bindemittel neben anderen fakultativen Komponenten.

Bevorzugte organische filmbildende Bindemittel sind Epoxidharze, Polyurethanharze, Polyesterharze, Acrylharze und deren Copolymerharze, Polyvinylharze, Phenolharze, Alkydharze oder Mischungen von solchen Harzen.

Als organisches filmbildendes Bindemittel der Ueberzugszusammensetzung sind alle üblichen Filmbildner für lösungsmittelhaltige, insbesondere jedoch für wässrige Lackzusammensetzungen geeignet. Beispiele für solche Filmbildner sind Epoxidharze, Polyurethanharze, Aminoplastharze oder Mischungen solcher Harze; eine basische wässrige Dispersion oder eine Lösung eines sauren Harzes.

Von besonderem Interesse sind organische filmbildende Bindemittel für wässrige Ueberzugszusammensetzungen wie z.B. Alkydharze; Acrylharze; 2-Komponenten-Epoxidharze; Polyurethanharze; Polyesterharze, welche üblicherweise gesättigt sind; wasserverdünnbare Phenolharze oder abgeleitete Dispersionen; wasserverdünnbare Harnstoffharze; Harze auf Basis von Vinyl-/Acrylcopolymeren.

Spezifischer betrachtet können die Alkydharze wasserverdünnbare Alkydharzsysteme sein, welche lufttrocknend oder in Form von Einbrennsystemen wahlweise in Kombination mit wasserverdünnbaren Melaminharzen eingesetzt werden können; es kann sich auch um oxidativ trocknende, lufttrocknende oder Einbrennsysteme handeln, welche wahlweise in Kombination mit wässrigen Dispersionen auf Basis von Acrylharzen oder deren Copolymeren, mit Vinylacetaten etc. angewandt werden.

Die Acrylharze können reine Acrylharze, Acrylsäureestercopolymere, Kombinationen mit Vinylharzen oder Copolymere mit Vinylmonomeren wie Vinylacetat, Styrol oder Butadien sein. Diese Systeme können lufttrocknende Systeme oder Einbrennsysteme sein.

Wasserverdünnbare Epoxidharze weisen in Kombination mit geeigneten Polyaminvernetzern ausgezeichnete mechanische und chemische Beständigkeit auf. Bei Verwendung von flüssigen Epoxidharzen kann auf einen Zusatz organischer Lösungsmittel zu wässrigen Systemen verzichtet werden. Die Anwendung von Festharzen oder Festharzdispersionen erfordert üblicherweise einen Zusatz geringfügiger Lösungsmittelmengen, um die Filmbildung zu verbessern.

Bevorzugte Epoxidharze sind solche auf Basis aromatischer Polyole, insbesondere auf Basis von Bisphenolen. Die Epoxidharze werden in Kombination mit Vernetzern angewandt. Bei letzteren kann es sich um insbesondere amino- oder hydroxyfunktionelle Verbindungen, eine Säure, ein Säureanhydrid oder eine Lewis-Säure handeln. Beispiele dafür sind Polyamine, Polyaminoamide, Polymere auf Basis von Polysulfiden, Polyphenole, Borfluoride und deren Komplexverbindungen, Polycarbonsäuren, 1 ,2-Dicarbonsäureanhydride oder Pyromellitsäuredianhydrid.

Polyurethanharze leiten sich von Polyethern, Polyestern und Polybutadienen mit endständigen Hydroxylgruppen einerseits und aliphatischen oder aromatischen Polyisocyanaten andererseits ab.

Geeignete Polyvinylharze sind beispielsweise Polyvinylbutyral, Polyvinylacetat oder deren Copolymere.

Geeignete Phenolharze sind Kunstharze, bei deren Aufbau Phenole die Hauptkomponente darstellen, also vorallem Phenol-, Kresol-, Xylenol- und Resorcin-Formaldehyd-Harze, Alkylphenolharze sowie Kondensationsprodukte aus Phenolen mit Acetaldehyd, Furfurol, Acrolein oder anderen Aldehyden. Von Interesse sind auch modifizierte Phenolharze.

Die Ueberzugszusammensetzungen können zusätzlich eine oder mehrere Komponenten aus der Gruppe der Pigmente, Farbstoffe, Füllstoffe, Fliesskontrollmittel, Dispergiermittel, Thixotropiemittel, Haftungsverbesserer, Antioxidantien, Lichtstabilisatoren oder Härtungskatalysatoren enthalten. Sie können auch noch andere bekannte Korrosionsschutzmittel enthalten, beispielsweise Korrosionsschutz-Pigmente, wie phosphat- oder borathaltige Pigmente oder Metalloxid-Pigmente, oder andere organische oder anorganische Korrosionsinhibitoren, z.B. Salze der Nitroisophthalsäure, Phosphorester, technische Amine oder substituierte Benztriazole.

Die Pigmente sind beispeilsweise Titandioxid, Eisenoxid, Aluminiumbronze oder Phthalocyaninblau.

Beispiele für Füllstoffe sind Talk, Aluminiumoxid, Aluminiumsilikat, Baryt, Glimmer oder Siliciumdioxid. Die erfindungsgemässen Korrosionsinhibitoren können auch auf einen Trägerstoff aufgebracht werden. Hierfür eignen sich insbesondere pulverförmige Füllstoffe oder Pigmente.

Fliesskontrollmittel und Thixotropiemittel basieren auf modifizierten Bentoniten.

Haftungsverbesserer basieren auf modifizierten Silanen.

Von Vorteil ist ferner der Zusatz von basischen Füllstoffen oder Pigmenten, die in bestimmten Bindemittelsystemen einen synergistischen Effekt auf die Korrosionsinhibierung bewirken. Beispiele für solche basischen Füllstoffe und Pigmente sind Calcium- oder Magnesiumcarbonat, Zinkoxid, Zinkcarbonat, Zinkphosphat, Magnesiumoxid, Aluminiumoxid, Aluminiumphosphat oder Gemische davon. Beispiele für basische organische Pigmente sind solche auf Basis von Aminoanthrachinon.

Die erfindungsgemässen Korrosionsinhibitoren können dem Anstrichmittel während dessen Herstellung zugesetzt werden, beispielsweise während der Pigmentverteilung durch Mahlen, oder der Inhibitor wird in einem Lösungsmittel gelöst und anschliessend in die Ueberzugszusammensetzung eingerührt. Die erfindungsgemässen Korrosionsinhibitoren können ebenso zur Vorbehandlung der Metalloberfläche verwendet werden.

Die erfindungsgemässen Erdalkalimetallsalze, Uebergangsmetallsalze und Uebergangsmetallkomplexe von Verbindungen der Formel I werden zweckmässig in einer Menge von 0,01 bis 20 Gew.-%, vorzugsweise 0,05 bis 5 Gew.-%, bezogen auf das Gesamtgewicht der Ueberzugszusammensetzung, verwendet.

Die Anstrichmittel können nach den üblichen Verfahren auf das Substrat aufgebracht werden, beispielsweise durch Sprühen, Tauchen, Streichen oder durch Elektroabscheidung. Oft werden mehrere Schichten aufgetragen. Die Korrosionsinhibitoren werden in erster Linie der Grundschicht (Primer) zugegeben, da sie vor allem an der Grenze Metall-Anstrich wirken. Sie können aber auch zusätzlich zur Zwischen- oder Deckschicht zugegeben werden. Je nachdem, ob das Bindemittel ein physikalisch, chemisch oder oxidativ trocknendes Harz oder ein hitze- oder strahlenhärtendes Harz ist, erfolgt die Härtung des Anstrichs bei Raumtemperatur oder durch Erwärmen (Einbrennen) oder durch Bestrahlung.

Vorzugsweise ist das Anstrichmittel ein Grundanstrich (Primer) für metallische Substrate, wie beispielsweise Eisen, Stahl, Kupfer, Zink oder Aluminium, sowie deren Legierungen.

Zusätzlich zur antikorrosiven Wirkung haben die erfindungsgemässen Erdalkalimetallsalze und Uebergangsmetallkomplexe von Verbindungen der Formel I den Vorteil, dass sie die Adhäsion Anstrich-Metall günstig beeinflussen und keine negativen Auswirkungen auf die Lagerstabilität der erfindungsgemässen Uebergangszusammensetzungen zeigen.

Eine bevorzugte Ausführungsform der vorliegenden Erfindung ist daher die Verwendung der Erdalkalimetallsalze, Uebergangsmetallsalze und Uebergangsmetallkomplexe von Verbindungen der Formel I als Korrosionsinhibitoren in Ueberzugszusammensetzungen für metallische Oberflächen.

Die vorliegende Erfindung betrifft auch ein Verfahren zum Schutz eines korrodierbaren Metallsubstrats, das dadurch gekennzeichnet ist, dass man auf dieses eine Uebergangszusammensetzung aufbringt, die a) ein organisches filmbildendes Bindemittel und b) als Korrosionsinhibitor mindestens ein Erdalkalimetallsalz, ein Uebergangsmetallsalz oder einen Uebergangsmetallkomplex von Verbindungen der Formel I enthält und sie anschliessend trocknet und/oder härtet.

Die folgenden Beispiele erläutern die Erfindung weiter. Angaben in Teilen oder Prozenten beziehen sich auf das Gewicht.

### Beispiel 1: Herstellung des Zinksalzes der 3-(4-Methylbenzoyl)propionsäure (Verbindung (101), Tabelle 1).

Zu einer Lösung von 38,4 g (0,20 Mol) 3-(4-Methylbenzoyl)propionsäure in 200 ml 1,0 N Natriumhydroxid-Lösung wird unter gutem Rühren eine Lösung von 59,5 g (0,20 Mol) Zinknitrat Hexahydrat [Zn(NO₃)₂6H₂O] in 200 ml Wasser getropft. Der Niederschlag wird filtriert, mit Wasser mehrmals gut gewaschen und im Vakuumschrank bei 50°C getrocknet. Es resultiert das Zinksalz der 3-(4-Methylbenzoyl)propionsäure, Smp. ∼220°C (Verbindung (101), Tabelle 1).

In Analogie zu Beispiel 1 werden aus dem Mangannitrat, Cobaltnitrat, Cernitrat, Eisennitrat, Lanthanchlorid, Yttriumchlorid und Calciumnitrat mit der 3-(4-Methylbenzoyl)propionsäure die Verbindungen (102) bis (108) hergestellt (vgl. Tabelle 1).

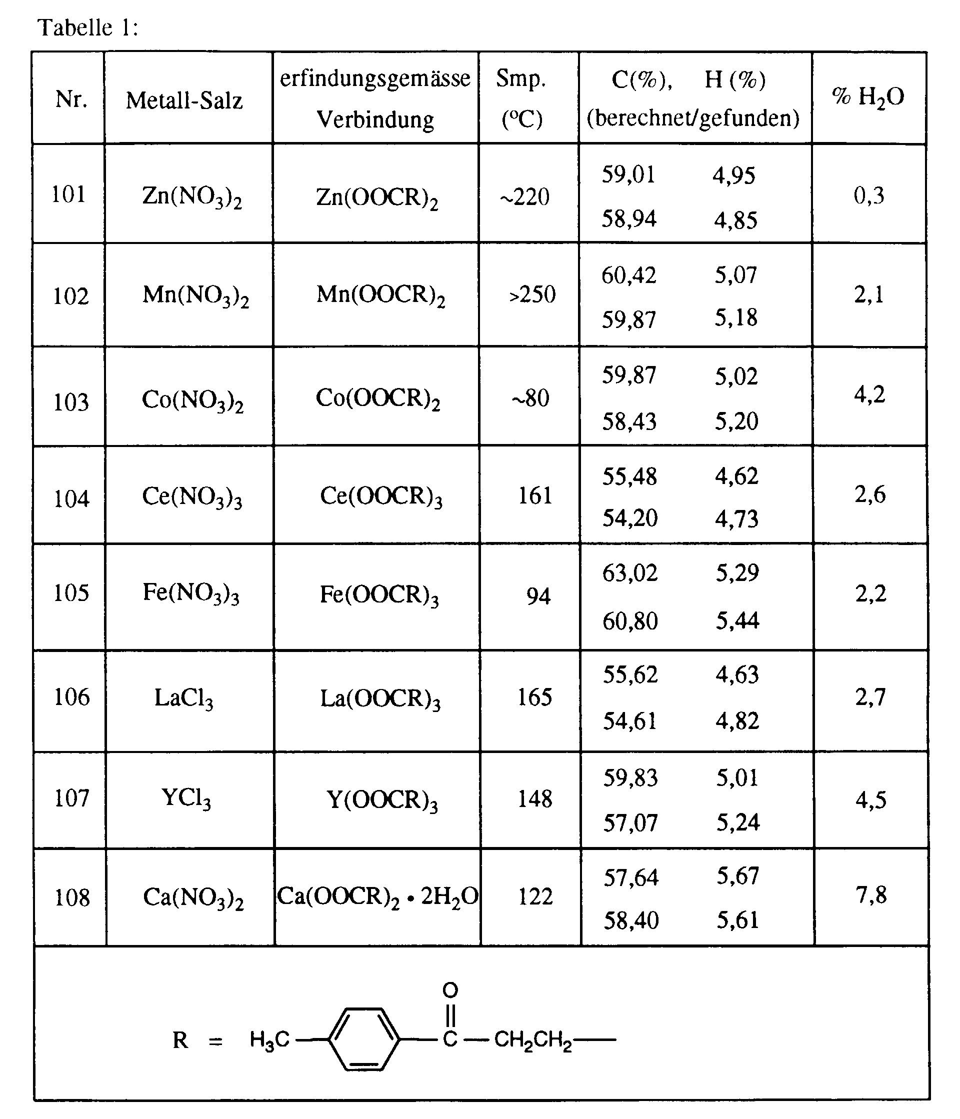

Die Definition von R in Tabelle 1 gilt auch für die Beispiele 2 bis 14 und 16.

### Beispiel 2: Herstellung des Zirkoniumkomplexes der 3-(4-Methylbenzoyl)propionsäure (Verbindung (109)) mit Zirkoniumsulfat.

Zu einer Lösung von 19,2 g (0,10 Mol) 3-(4-Methylbenzoyl)propionsäure in 100 ml 1,0 N Natriumhydroxid-Lösung wird unter gutem Rühren eine Lösung von 18,3 g (0,05 Mol) Zirkoniumsulfat (22,8 % Zirkonium) in 40 ml Wasser getropft. Der Niederschlag wird filtriert, mit Wasser mehrmals gut gewaschen und im Vakuumschrank bei 50°C getrocknet. Es resultiert der Zirkoniumkomplex der 3-(4-Methylbenzoyl)propionsäure (Verbindung (109)). Elementaranalyse berechnet für ZrO(OH)(O₂CR)·6,6 HO₂CR: Zr 5,8 %; C 63,4 %; H 5,8 %; HO₂CR 80,1 %. Gefunden: Zr 7,4 %; C 58,6 %; H 5,6 %; H₂O 2,3 %; HO₂CR 79,8 %.

### Beispiel 3: Herstellung des Zirkoniumkomplexes der 3-(4-Methylbenzoyl)propionsäure (Verbindung (110)) mit Zirkoniumsulfat.

Zu einer Lösung von 76,9 g (0,40 Mol) 3-(4-Methylbenzoyl)propionsäure in 400 ml 1,0 N Natriumhydroxid-Lösung wird unter gutem Rühren eine Lösung von 35,5 g (0,089 Mol) Zirkoniumsulfat (22,8 % Zirkonium) in 70 ml Wasser getropft. Der Niederschlag wird filtriert, mit Wasser mehrmals gut gewaschen und im Vakuumschrank bei 50°C getrocknet. Es resultiert der Zirkoniumkomplex der 3-(4-Methylbenzoyl)propionsäure (Verbindung (110)). Elementaranalyse berechnet für ZrO(OH)(O₂CR)·2,9 HO₂CR: Zr 10,4 %; C 59,0 %; H 5,4 %; HO₂CR 63,8 %. Gefunden: Zr 9,4 %; C 58,3 %; H 5,4 %; H₂O 2,0 %; HO₂CR 64,2 %.

### Beispiel 4: Herstellung des Zirkoniumkomplexes der 3-(4-Methylbenzoyl)propionsäure (Verbindung (111)) mit Zirkoniumoxidchlorid.

Zu einer Lösung von 57,7 g (0,30 Mol) 3-(4-Methylbenzoyl)propionsäure in 300 ml 1,0 N Natriumhydroxid-Lösung wird unter gutem Rühren eine Lösung von 28,1 g (0,115 Mol) Zirkoniumoxidchlorid (37,2 % Zirkonium) in 55 ml Wasser getropft. Der Niederschlag wird filtriert, mit Wasser mehrmals gut gewaschen und im Vakuumschrank bei 50°C getrocknet. Es resultiert der Zirkoniumkomplex der 3-(4-Methylbenzoyl)propionsäure (Verbindung (111)). Elementaranalyse berechnet für ZrO(OH)(O₂CR)·0,3 HO₂CR: Zr 24,5 %; C 46,0 %; H 4,2 %; HO₂CR 15,5 %. Gefunden: Zr 25,2 %; C 48,8 %; H 4,6 %; H₂O 3,2 %; HO₂CR 8,7 %.

### Beispiel 5: Herstellung des Zirkoniumkomplexes der 3-(4-Methylbenzoyl)propionsäure (Verbindung (112)) mit Zirkoniumoxidchlorid.

Zu einer Lösung von 19,2 g (0,10 Mol) 3-(4-Methylbenzoyl)propionsäure in 100 ml 1,0 N Natriumhydroxid-Lösung wird unter gutem Rühren eine Lösung von 14,3 g (0,058 Mol) Zirkoniumoxidchlorid (37,2 % Zirkonium) in 30 ml Wasser getropft. Der Niederschlag wird filtriert, mit Wasser mehrmals gut gewaschen und im Vakuumschrank bei 50°C getrocknet. Es resultiert der Zirkoniumkomplex der 3-(4-Methylbenzoyl)propionsäure (Verbindung (112)). Elementaranalyse berechnet für ZrO(OH)(O₂CR)·0,7 HO₂CR: Zr 20,3 %; C 49,9 %; H 4,6 %; HO₂CR 29,9 %. Gefunden: Zr 19,2 %; C 48,3 %; H 4,7 %; H₂O 5,5 %; HO₂CR 40,0 %.

### Beispiel 6: Herstellung des Zirkoniumkomplexes der 3-(4-Methylbenzoyl)propionsäure (Verbindung (113)) mit Zirkoniumacetat.

Zu einer Lösung von 19,2 g (0,10 Mol) 3-(4-Methylbenzoyl)propionsäure in 100 ml 1,0 N Natriumhydroxid-Lösung wird unter gutem Rühren eine Lösung von 37,2 g (0,075 Mol) Zirkoniumacetat (18,4 % Zirkonium) in 60 ml Wasser getropft. Der Niederschlag wird filtriert, mit Wasser mehrmals gut gewaschen und im Vakuumschrank bei 50°C getrocknet. Es resultiert der Zirkoniumkomplex der 3-(4-Methylbenzoyl)propionsäure (Verbindung (113)). Elementaranalyse berechnet für ZrO(OH)(O₂CR)_{0,8}(Acetat)_{0,2}: Zr 31,6 %; C 38,2 %; H 3,6 %; Acetat 4,1 %. Gefunden: Zr 32,6 %; C 38,6 %; H 4,1 %; H₂O 2,5 %; Acetat 5,7 %.

### Beispiel 7: Herstellung des Zirkoniumkomplexes der 3-(4-Methylbenzoyl)propionsäure (Verbindung (114)) mit Zirkoniumacetat.

Zu einer Lösung von 9,60 g (0,05 Mol) 3-(4-Methylbenzoyl)propionsäure in 50 ml 1,0 N Natriumhydroxid-Lösung wird unter gutem Rühren eine Lösung von 148,8 g (0,30 Mol) Zirkoniumacetat (18,4 % Zirkonium) in 100 ml Wasser getropft. Der Niederschlag wird filtriert, mit Wasser mehrmals gut gewaschen und im Vakuumschrank bei 50°C getrocknet. Es resultiert der Zirkoniumkomplex der 3-(4-Methylbenzoyl)propionsäure (Verbindung (114)). Elementaranalyse berechnet für ZrO(OH)(O₂CR)_{0,5}(Acetat)_{0,5}: Zr 36,6 %; C 31,3 %; H 3,2 %; Acetat 11,8 %. Gefunden: Zr 34,9 %; C 29,7 %; H 3,8 %; H₂O 3,2 %; Acetat 12,8 %.

### Beispiel 8: Herstellung des Zirkoniumkomplexes der 3-(4-Methylbenzoyl)propionsäure (Verbindung (115)) mit Zirkoniumcarbonat.

Eine Suspension von 15,0 g (56,0 mMol) Zirkoniumcarbonat (34 % Zirkonium) und 15,0 g (78,0 mMol) 3-(4-Methylbenzoyl)propionsäure in 150 ml Wasser wird unter intensivem Rühren innerhalb 2 Stunden auf 90°C erwärmt. Anschliessend wird das Reaktionsgemisch abgekühlt, das Wasser abdekantiert und der Rückstand mit Essigsäureethylester extrahiert. Das Lösungsmittel wird am Vakuumrotationsverdampfer eingeengt und der Rückstand am Hochvakuum bei 50°C getrocknet. Es resultiert der Zirkoniumkomplex der 3-(4-Methylbenzoyl)propionsäure (Verbindung (115)). Elementaranalyse berechnet für ZrO(OH)(O₂CR)·0,3 HO₂CR: Zr 24,5 %; C 46,0 %; H 4,2 %; HO₂CR 15,5 %. Gefunden: Zr 21,0 %; C 48,1 %; H 4,6 %; H₂O 0,4 %; HO₂CR 16,5 %.

### Beispiel 9: Herstellung des Zirkoniumkomplexes der 3-(4-Methylbenzoyl)propionsäure (Verbindung (116)) mit Zirkoniumcarbonat.

Eine Suspension von 45,0 g (168 mMol) Zirkoniumcarbonat (34 % Zirkonium) und 15,0 g (78,0 mMol) 3-(4-Methylbenzoyl)propionsäure in 150 ml Wasser wird unter intensivem Rühren innerhalb 2 Stunden auf 90°C erwärmt. Anschliessend wird das Reaktionsgemisch abgekühlt, das Wasser abdekantiert, der Rückstand mit Essigsäureethylester extrahiert und der Rückstand am Hochvakuum bei 50°C getrocknet. Es resultiert der Zirkoniumkomplex der 3-(4-Methylbenzoyl)propionsäure (Verbindung (116)). Elementaranalyse berechnet für ZrO₂·ZrO(OH)(O₂CR): Zr 41,6 %; C 30,1 %; H 4,6 %. Gefunden: Zr 37,7 %; C 26,8 %; H 3,7 %; H₂O 2,7 %.

### Beispiel 10: Herstellung des Zirkoniumkomplexes der 3-(4-Methylbenzoyl)propionsäure (Verbindung (117)) mit Zirkonium-n-propoxid.

Eine Lösung von 44,3 g (0,10 Mol) Zirkonium-n-propoxid (20,6 % Zirkonium) und 19,2 g (0,10 Mol) 3-(4-Methylbenzoyl)propionsäure in 200 ml trockenem Toluol wird unter Stickstoffatmosphäre während 12 Stunden bei 50°C gerührt. Anschliessend wird das Reaktionsgemisch abgekühlt und am Vakuumrotationsverdampfer eingeengt und der Rückstand am Hochvakuum bei 50°C getrocknet. Es resultiert der Zirkoniumkomplex der 3-(4-Methylbenzoyl)propionsäure (Verbindung (117)). Elementaranalyse berechnet für Zr(OC₃H₇)₃(O₂CR): Zr 19,8 %; C 52,2 %; H 7,03 %. Gefunden: Zr 20,0 %; C 52,4 %; H 6,75 %.

### Beispiel 11: Herstellung des Zirkoniumkomplexes der 3-(4-Methylbenzoyl)propionsäure (Verbindung (118)) mit Zirkoniumcarbonat.

Eine Suspension von 50 g (0,18 Mol) Zirkoniumcarbonat (32,8 % Zirkonium) und 50 g (0,26 Mol) 3-(4-Methylbenzoyl)propionsäure in 500 ml Wasser und 500 ml Toluol wird unter intensivem Rühren innerhalb von 30 Minuten auf 83°C erwärmt. Das Reaktionsgemisch wird anschliessend noch während 45 Minuten bei 83°C weitergerührt. Die noch warme organische Phase wird abgetrennt und am Vakuumrotationsverdampfer auf ein Restvolumen von 200 ml eingeengt. Anschliessend wird die Lösung unter intensivem Rühren in 2000 ml Benzin gegeben. Der dabei entstehende Niederschlag wird abfiltriert und am Hochvakuum bei 50°C getrocknet. Es resultiert der Zirkoniumkomplex der 3-(4-Methylbenzoyl)propionsäure (Verbindung (118)). Elementaranalyse berechnet für ZrO(OH)(O₂CR)·0,48 HO₂CR: Zr 22,4 %; C 47,9 %; H 4,4 %; HO₂CR 22,6 %. Gefunden: Zr 22,2 %; C 47,7 %; H 4,8 %; H₂O 0,75 %; HO₂CR 20,0 %.

### Beispiel 12: Herstellung des Zirkoniumkomplexes der 3-(4-Methylbenzoyl)propionsäure (Verbindung (119)) mit Zirkonium-n-propoxid.

Eine Lösung von 44,3 g (0,10 Mol) Zirkonium-n-propoxid (20,6 % Zirkonium) und 38,4 g (0,20 Mol) 3-(4-Methylbenzoyl)propionsäure in 200 ml trockenem Toluol wird unter Stickstoffatmosphäre während 12 Stunden bei 50°C gerührt. Anschliessend wird das Reaktionsgemisch abgekühlt, am Vakuumrotationsverdampfer eingeengt und der Rückstand am Hochvakuum bei 50°C getrocknet. Es resultiert der Zirkoniumkomplex der 3-(4-Methylbenzoyl)propionsäure (Verbindung (119)). Elementaranalyse berechnet für Zr(OC₃H₇)₂(O₂CR)₂: Zr 15,4 %; C 56,8 %; H 6,1 %. Gefunden: Zr 15,1 %; C 56,7 %; H 6,2 %.

### Beispiel 13: Herstellung des Zirkoniumkomplexes der 3-(4-Methylbenzoyl)propionsäure (Verbindung (120)) mit Zirkonium-n-butoxid.

Eine Lösung von 43,2 g (0,10 Mol) Zirkonium-n-butoxid (21,1 % Zirkonium) und 19,2 g (0,10 Mol) 3-(4-Methylbenzoyl)propionsäure in 200 ml trockenem Toluol wird unter Stickstoffatmosphäre während 12 Stunden bei 50°C gerührt. Anschliessend wird das Reaktionsgemisch abgekühlt, am Vakuumrotationsverdampfer eingeengt und der Rückstand am Hochvakuum bei 50°C getrocknet. Es resultiert der Zirkoniumkomplex der 3-(4-Methylbenzoyl)propionsäure (Verbindung (120)). Elementaranalyse berechnet für Zr(OC₄H₉)₃(O₂CR): Zr 18,2 %; C 55,0 %; H 7,6 %. Gefunden: Zr 19,3 %; C 54,2 %; H 7,0 %.

### Beispiel 14: Herstellung des Zirkoniumkomplexes der 3-(4-Methylbenzoyl)propionsäure (Verbindung (121)) mit Zirkonium-i-propoxid.

Eine Lösung von 17,3 g (0,05 Mol) Zirkonium-i-propoxid (26,4 % Zirkonium) und 9,6 g (0,05 Mol) 3-(4-Methylbenzoyl)propionsäure in 200 ml trockenem Toluol wird unter Stickstoffatmosphäre während 12 Stunden bei 50°C gerührt. Anschliessend wird das Reaktionsgemisch abgekühlt, am Vakuumrotationsverdampfer eingeengt und der Rückstand am Hochvakuum bei 50°C getrocknet. Es resultiert der Zirkoniumkomplex der 3-(4-Methylbenzoyl)propionsäure (Verbindung (121)). Elementaranalyse berechnet für Zr(OC₃H₇)₃(O₂CR): Zr 19,8 %; C 52,2 %; H 7,0 %. Gefunden: Zr 23,8 %; C 47,4 %; H 5,8 %.

### Beispiel 15: Herstellung des Zirkoniumkomplexes der 3-(4-Chlorbenzoyl)propionsäure (Verbindung (122)) mit Zirkoniumcarbonat.

Eine Suspension von 30 g (0,108 Mol) Zirkoniumcarbonat (32,8 % Zirkonium) und 33,2 g (0,156 Mol) 3-(4-Chlorbenzoyl)propionsäure in 300 ml Wasser wird unter intensivem Rühren innerhalb von 30 Minuten auf 90°C erwärmt. Das Reaktionsgemisch wird anschliessend noch während 45 Minuten bei 90°C weitergerührt. Dann wird das Wasser heiss abdekantiert und der Rückstand mit Essigsäureethylester extrahiert. Das Lösungsmittel wird am Vakuumrotationsverdampfer eingeengt und der Rückstand am Hochvakuum bei 50°C getrocknet. Es resultiert der Zirkoniumkomplex der 3-(4-Chlorbenzoyl)propionsäure (Verbindung (122)). Elementaranalyse berechnet für ZrO(OH)(O₂CR') ·0,47 HO₂CR': Zr 20,9 %; C 40,5 %; H 3,1 %; Cl 12,0 %; HO₂CR' 22,9 %. Gefunden: Zr 20,7 %; C 40,1 %; H 3,3 %; Cl 11,6 %; H₂O 0,6 %; HO₂CR' 29,7 %.

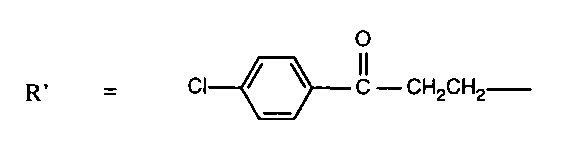

### Beispiel 16: Herstellung des Titankomplexes der 3-(4-Methylbenzoyl)propionsäure (Verbindung (123)) mit Titan(IV)-n-propoxid.

Eine Lösung von 14,2 g (50 mMol) Titan(IV)-n-propoxid und 9,61 g (50 mMol) 3-(4-Methylbenzoyl)propionsäure in 100 ml trockenem Toluol wird unter Stickstoffatmosphäre während 12 Stunden bei 50°C gerührt. Anschliessend wird das Reaktionsgemisch abgekühlt und am Vakuumrotationsverdampfer eingeengt und der Rückstand am Hochvakuum bei 50°C getrocknet. Es resultiert der Titankomplex der 3-(4-Methylbenzoyl)propionsäure (Verbindung (123)). Elementaranalyse berechnet für Ti(OC₃H₇)₃(O₂CR): Ti 11,5 %; C 57,7 %; H 7,8 %. Gefunden: Ti 11,5 %; C 56,5 %; H 7,6 %.

### Beispiel 17: Prüfung von Erdalkalimetallsalzen, Uebergangsmetallsalzen und Uebergangsmetallkomplexen der 3-(4-Methylbenzoyl)propionsäure in Acryldispersionen auf Basis Maincote HG-54 als Korrosionsinhibitoren.

Zur Herstellung der Ueberzugszusammensetzung auf Basis Maincote HG-54 werden die Komponenten 1 bis 8 (Formulierung ohne Additive) bzw. die Komponenten 1 bis 9 (Formulierung mit Additiv) in der angegebenen Reihenfolge eingesetzt (vgl. Tabelle 2).

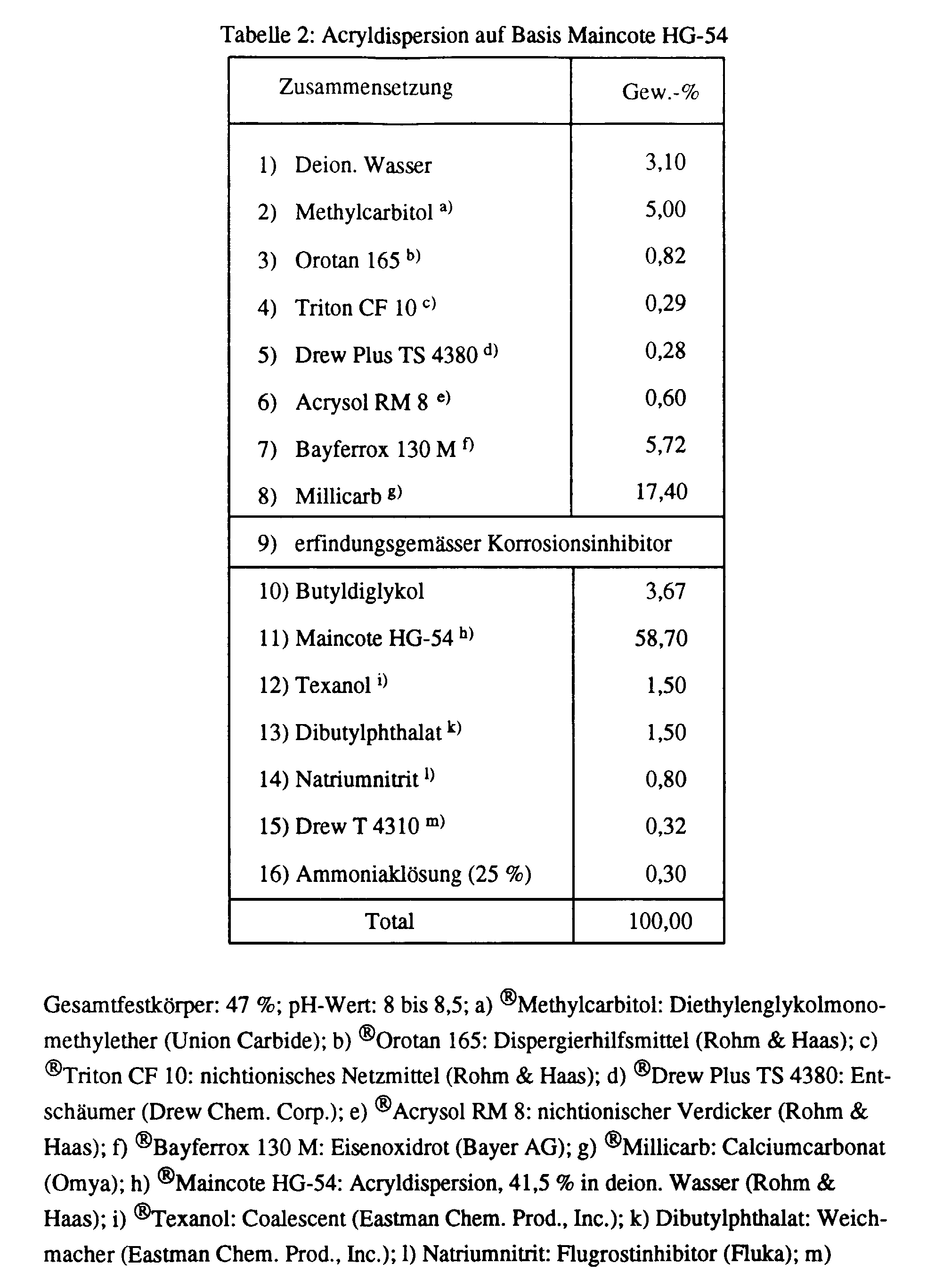

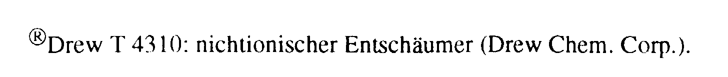

Die Komponenten werden unter Verwendung eines Schnellrührers bei 3000 Umdrehungen/Minute auf eine Mahlfeinheit bzw. Mahlkörnigkeit von < 15 µm dispergiert. Das Dispergierergebnis der so erhaltenen Pigmentpaste wird duch Ermittlung des Grindometerwertes (ISO 1524) beurteilt. Die Einsatzmenge der erfindungsgemässen Korrosionsinhibitoren wird auf den Gesamtfestkörper der Formulierung ohne Additiv (Gesamtfestkörper: 47 %) bezogen. Demzufolge bedeutet beispielsweise Zusatz von 1 % Korrosionsinhibitor in 100 g Dispersion eine Menge von 0,47 g. Zur Fertigstellung der Ueberzugszusammensetzung werden die Komponenten 10 bis 16 gemäss Tabelle 2 bei reduzierter Rührgeschwindigkeit (1000 Umdrehungen/Minute) in der angegebenen Reihenfolge zugegeben. Anschliessend wird der pH-Wert der Formulierung kontrolliert und vor der Applikation gegebenenfalls mit Ammoniaklösung (25 %ig) auf einen Wert von pH 8 bis 8,5 nachgestellt.

Die Applikation der Ueberzugszusammensetzung kann unverdünnt durch Airless-Spritzen oder nach Verdünnung durch Streichen, Rollen oder konventionelles Spritzen erfolgen. Zur Applikation durch konventionelles Spritzen werden die Formulierungen auf eine Spritzviskosität von 22 bis 23 Sekunden (Ford-Becher 4; DIN 53 211) verdünnt. Verdünner: Butylglykol/deion. Wasser = 1:1 (g/g).

Die Applikation der Formulierung erfolgt auf Stahlbleche (10 mal 15 cm) des Typs Q-Panel R (kalt gewalzter, unbehandelter Stahl; Hersteller: The Q-Panel Company, Cleveland, USA) in einer Schichtdicke, welche nach dem Trocknen 50 µm beträgt (Trocknungsbedingungen: 10 Tage bei Raumtemperatur).

Vor Bewitterungsbeginn wird an den "Lackfilmen" unter Verwendung eines BonderKreuzschnittgeräts (Mod. 205; Hersteller/Vertrieb: Firma Lau, 5870 Hemer/Deutschland) eine definierte Verletzung (70 mal 0,5 mm) in Form eines Parallelschnitts (d.h. parallel zur längsten Blechkante) angebracht. Die Blechkanten werden durch Anbringen eines Kantenschutzes (®Icosit 255; Hersteller: Inertol AG, Winterthur, Schweiz) geschützt.

Die Proben werden im Anschluss einer Schnellbewitterung im Salzsprühtest (DIN 50 021 SS) während 168 Stunden und im Schwitzwassertest (ASTM D 4585-87) während 330 Stunden unterzogen. Die Resultate sind in Tabelle 3 bis 6 zusammengefasst. Die Bewertung der Resultate erfolgt basierend auf den relevanten DIN-Normen nach einem Bewertungsschlüssel durch Angabe eines Korrosionsschutzwertes CPF ("**C**orrosion **P**rotection **F**actor). Der CPF setzt sich additiv aus einer Beurteilung des Anstrichs (Film) und einer Beurteilung des Stahls zusammen und beträgt im Maximum 12 Punkte. Die Einzel-Maximalwerte für den Anstrich (Film) und den Stahl betragen 6 Punkte. Je grösser die Zahlen sind, desto besser ist der Korrosionsschutz.

**Tabelle 3:**

| Salzsprühtest, 168 Stunden | | | |
|---|---|---|---|
| Verbindung | CPF Film | CPF Metall | CPF |
| ― | 2,8 | 3,0 | 5,8 |
| 1 % (102) | 3,6 | 3,6 | 7,2 |
| 2 % (102) | 4,0 | 4,5 | 8,5 |
| 1 % (104) | 4,0 | 4,5 | 8,5 |
| 2 % (104) | 4,0 | 5,5 | 9,5 |
| 1 % (105) | 3,2 | 4,5 | 7,7 |
| 2 % (105) | 4,0 | 4,5 | 8,5 |
| 1 % (111) | 4,0 | 5,0 | 9,0 |
| 2 % (111) | 4,0 | 5,0 | 9,0 |
| 1 % (112) | 3,2 | 4,8 | 8,0 |
| 2 % (112) | 4,0 | 4,8 | 8,8 |
| 1 % (113) | 4,0 | 5,5 | 9,5 |
| 2 % (113) | 4,0 | 5,3 | 9,3 |
| 1 % (114) | 3,4 | 4,8 | 8,2 |
| 2 % (114) | 3,8 | 5,5 | 9,3 |
| 1 % (116) | 3,4 | 4,0 | 7,4 |
| 2 % (116) | 3,4 | 5,0 | 8,4 |

**Tabelle 4:**

| Salzsprühtest, 168 Stunden | | | |
|---|---|---|---|
| Verbindung | CPF Film | CPF Metall | CPF |
| ― | 2,4 | 2,3 | 4,7 |
| 1 % (122) | 3,0 | 4,7 | 7,7 |
| 2 % (122) | 3,2 | 5,4 | 8,6 |

**Tabelle 5:**

| Schwitzwassertest, 330 Stunden | | | |
|---|---|---|---|
| Verbindung | CPF Film | CPF Metall | CPF |
| ― | 3,2 | 2,0 | 5,2 |
| 1 % (107) | 3,4 | 5,5 | 8,9 |

**Tabelle 6:**

| Schwitzwassertest, 330 Stunden | | | |
|---|---|---|---|
| Verbindung | CPF Film | CPF Metall | CPF |
| ― | 2,8 | 2,0 | 4,8 |
| 1 % (122) | 3,2 | 5,8 | 9,0 |

### Beispiel 18: Prüfung von Erdalkalimetallsalzen, Uebergangsmetallsalzen und Uebergangsmetallkomplexen der 3-(4-Methylbenzoyl)propionsäure in Alkydharzsystemen auf Basis Bayhydrol B 130 H als Korrosionsinhibitoren.

Analog wie in Beispiel 17 beschrieben, werden zur Herstellung der Ueberzugszusammensetzung auf Basis Bayhydrol B 130 H die Komponenten 1 bis 6 (Formulierung ohne Additive) bzw. die Komponenten 1 bis 7 (Formulierung mit Additiv) in der angegeben Reihenfolge eingesetzt (vgl. Tabelle 7).

**Tabelle 7:**

| Alkyldharzformulierung auf Basis Bayhydrol B 130 H | |
|---|---|
| Zusammensetzung | Gew.-% |
| 1) Bayhydrol B 130 H ^{a)} | 60,03 |
| 2) Servosyn WEB (8 %) ^{b)} | 0,14 |
| 3) Ascinin R ^{c)} | 0,28 |
| 4) Bayferrox 130 M ^{d)} | 21,13 |
| 5) Heladol 10 ^{e)} | 5,15 |
| 6) Mikrotalk AT Extra ^{f)} | 10,57 |

| 7) erfindungsgemässer Korrosionsinhibitor | |
|---|---|
| 8) Aerosil 300 ^{g)} | 0,20 |
| 9) Zinkoxid | 1,06 |
| 10) Butylglykol | 0,90 |
| 11) Aluminiumoctoat | 0,05 |
| 12) Deion. Wasser | 0,49 |
| Total | 100,00 |
| Gesamtfestkörper: 56,1 %; | |

| | |
|---|---|
| a) ®Bayhydrol B 130 H; 30 % in deion. Wasser, Alkydharz, wasserverdünnbar (Bayer AG); | |
| b) ®Servosyn WEM (8 %): Kolbalt-Trockner (8 % Metall) (Servo Delden B.V.); | |
| c) ®Ascinin R: Hautverhinderungsmittel auf Oximbasis (Bayer AG); | |
| d) ®Bayferrox 130 M: Eisenoxidrot (Bayer AG); | |
| e) ®Heladol 10: Calciumcarbonat (Lange); | |
| f) Mikrotalk AT Extra: mikronisierter Talk (Norwegian); | |
| g) ®Aerosil 300: Verdickungs- und Thixotropie-Mittel, chemisch reine Kieselsäure (Degussa). | |

Die Komponenten werden entweder unter Verwendung eines Schnellrührers bei 3000 Umdrehungen/Minute oder unter Verwendung z.B. einer Horizontalkugelmühle auf eine Mahlfeinheit bzw. Mahlkörnigkeit von < 15 µm dispergiert. Das Dispergierergebnis der so erhaltenen Pigmentpaste wird duch Ermittlung des Grindometerwertes (ISO 1524) beurteilt. Die Einsatzmenge der erfindungsgemässen Korrosionsinhibitoren wird auf den Gesamtfestkörper der Formulierung ohne Additiv (Geamtfestkörper: 56,1 %) bezogen. Demzufolge bedeutet beispielsweise Zusatz von 1 % Korrosionsinhibitor in 100 g Formulierung eine Menge von 0,56 g. Zur Fertigstellung der Ueberzugszusammensetzung werden die Komponenten 8 bis 12 gemäss Tabelle 7 bei reduzierter Rührgeschwindigkeit (1000 Umdrehungen/Minute) in der angegebenen Reihenfolge zugegeben.

Die Applikation der Formulierung auf Stahlbleche des Typs Q-Panel R, die Durchführung des Salzsprühtests (168 Stunden) sowie die Bestimmung der Korrosionsschutzwerte CPF erfolgt analog wie in Beispiel 17 beschrieben. Die Resultate sind in Tabelle 8 zusammengefasst. Je grösser die Zahlen sind, desto besser ist der Korrosionsschutz.

**Tabelle 8:**

| Salzsprühtest, 168 Stunden | | | |
|---|---|---|---|
| Verbindung | CPF Film | CPF Metall | CPF |
| ― | 3,2 | 1,3 | 4,5 |
| 1 % (102) | 3,4 | 3,0 | 6,4 |
| 2 % (102) | 4,8 | 3,1 | 7,9 |
| 1 % (103) | 3,4 | 3,0 | 6,4 |
| 2 % (103) | 3,4 | 4,3 | 7,7 |
| 1 % (108) | 3,0 | 3,3 | 6,6 |
| 2 % (108) | 3,6 | 3,6 | 7,2 |

### Beispiel 19: Prüfung von Erdalkalimetallsalzen, Uebergangsmetallsalzen und Uebergangsmetallkomplexen der 3-(4-Methylbenzoyl)propionsäure in wässriger Dispersion auf Basis eines Acrylsäureester/Styrol-Copolymeren (Acronal S 760) als Korrosionsinhibitor.

Zur Herstellung der Ueberzugszusammensetzung auf Basis Acronal S 760 werden die Komponenten 1 bis 5 zunächst vorgemischt, danach erfolgt die Zugabe der Komponenten 7 und 8 (Formulierung ohne Korrosionsinhibitor) bzw. 6 bis 8 (Formulierung mit Korrosionsinhibitor, Verbindung (115), Beispiel 8) in der angegebenen Reihenfolge (vgl. Tabelle 9).

**Tabelle 9:**

| Wässrige Dispersion auf Basis Acronal S 760 | |
|---|---|
| Zusammensetzung | Gew.-% |
| 1) Deion. Wasser | 8,20 |
| 2) Pigmentverteiler NL^{a)} | 0,15 |
| 3) Acronal S 760 (50 % Lieferform)^{b)} | 8,00 |
| 4) Shellsol D 60^{c)} | 1,00 |
| 5) Agitan 280^{d)} | 0,30 |
| 6) erfindungsgemässer Korrosionsinhibitor | - |
| 7) Millicarb^{e)} | 10,00 |
| 8) Bayferrox 130 M^{f)} | 8,00 |
| 9) Acronal S-760 (50 % Lieferform)^{b)} | 49,00 |
| 10) Agitan 280^{d)} | 0,30 |
| 11) Collacral PU 85/Butyldiglykol (1:3 g/g)^{g)} | 4,10 |
| 12) Deion. Wasser | 0.95 |
| Total | 100,00 |
| Gesamtfestkörper: 57 %; | |

| | |
|---|---|
| a) ®Pigmentverteiler NL: Dispergierhilfsmittel (BASF AG); | |
| b) ®Acronal S 760: Dispersion eines Acrylsäureester-Styrol-Copolymers (wässrige Dispersion, BASF AG); | |
| c) ®Shellsol D 60: Lackbenzin (aliphatisches Lösungsmittel, Shell); | |
| d) ®Agitan 280: Entgasungs- und Entschäumungsmittel (Münzing Chemie GmbH); | |
| e) ®Millicarb: Calciumcarbonat (Firma Omya); | |
| f) Bayferrox 130 M: Eisenoxidrot (Bayer AG); | |
| g) ®Collacral PU 85: Verdickungsmittel (BASF AG). | |

Die resultierende Pigmentpaste wird mit einer Horizontalkugelmühle oder vergleichbarem auf eine Kornfeinheit < 15 µm dispergiert. Die Kornfeinheit wird anhand des Grindometerwertes (ISO 1524) beurteilt.

Zur Fertigstellung des Lacks (Auflacken) werden anschliessend die Komponenten 9 bis 12 in der angegebenen Reihenfolge (Tabelle 9) zugegeben. Die Applikation erfolgt durch konventionelles Spritzen. Je nach gewünschter Viskosität kann der fertige Lack durch Zugabe von Butyldiglykol/deion. Wasser (1:1 g/g) verdünnt werden.

Die Applikation des Lacks erfolgt, wie in Beispiel 17 beschrieben, auf Stahlbleche des Typs Bonder (kalt gewalzter, entfetteter Stahl, Hersteller: Firma Chemetall, Frankfurt am Main) in einer Schichtdicke, welche nach dem Trocknen 100 µm beträgt (Trocknungsbedingungen: 14 Tage bei Raumtemperatur).

Die Durchführung des Salzsprühtests (168 Stunden) sowie die Bestimmung der Korrosionsschutzwerte CPF erfolgt analog wie in Beispiel 17 beschrieben. Die Resultate sind in Tabelle 10 zusammengefasst. Je grösser die Zahlen sind, desto besser ist der Korrosionsschutz.

**Tabelle 10:**

| Salzsprühtest, 168 Stunden | | | |
|---|---|---|---|
| Verbindung | CPF Film | CPF Metall | CPF |
| ― | 2,4 | 2,6 | 5,0 |
| 1 % (115) | 3,8 | 6,0 | 9,8 |
| 2 % (115) | 4,0 | 6,0 | 10,0 |

## Patentansprüche

1. Erdalkalimetallsalze, Uebergangsmetallsalze und Uebergangsmetallkomplexe von Verbindungen der Formel I 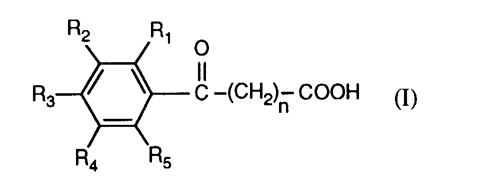 worin R₁, R₂, R₃, R₄ und R₅ unabhängig voneinander Wasserstoff, Halogen, Nitro, Cyano, CF₃, C₁-C₁₅-Alkyl, C₅-C₁₂-Cycloalkyl, C₂-C₁₅-Alkenyl, C₁-C₁₂-Halogenalkyl, C₁-C₁₂-Alkoxy, C₁-C₁₂-Alkylthio, unsubstituiertes oder durch C₁-C₄-Alkyl substituiertes C₆-C₁₀-Aryl; unsubstituiertes oder durch C₁-C₄-Alkyl substituiertes C₆-C₁₀-Aryloxy; unsubstituiertes oder am Arylrest durch 1 bis 3 C₁-C₄-Alkyl substituiertes C₇-C₁₂-Arylalkyl;
-CO₂R₆, -COR₆ oder 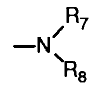 darstellen, wobei mindestens einer der Reste R₁ bis R₅
Wasserstoff, Halogen oder C₁-C₁₅-Alkyl bedeutet, ferner die Reste R₁ und R₂, R₂ und R₃, R₃ und R₄ oder R₄ und R₅ zusammen mit den Kohlenstoffatomen, an die sie gebunden sind, einen Benzo- oder Cyclohexenyl-Ring bilden,
R₆ C₁-C₂₀-Alkyl, durch Sauerstoff, Schwefel oder 〉N-R₉ unterbrochenes C₂-C₂₀-Alkyl; unsubstituiertes oder am Arylrest durch 1 bis 3 C₁-C₄-Alkyl substituiertes C₇-C₁₂-Arylalkyl bedeutet,
R₇ und R₈ unabhängig voneinander Wasserstoff, C₁-C₂₄-Alkyl oder durch Sauerstoff, Schwefel oder 〉N-R₉ unterbrochenes C₂-C₂₄-Alkyl darstellen,
R₉ Wasserstoff oder C₁-C₈-Alkyl bedeutet, und
n eine ganze Zahl aus dem Bereich von 1 bis 10 darstellt; mit der Bedingung, dass das Calciumsalz der 3-(3-Chlor-4-isopropyl-benzoyl)propionsäure, das Calciumsalz der 3-(3-Chlor-4-cyclohexyl-benzoyl)propionsäure und das Calcium-, Magnesium- und Zinksalz der 4-Benzoylbuttersäure ausgeschlossen sind.

2. Verbindungen gemäss Anspruch 1, worin
R₁, R₂, R₃, R₄ und R₅ unabhängig voneinander Wasserstoff, Halogen, Nitro, Cyano, CF₃, C₁-C₁₅-Alkyl, C₅-C₁₂-Cycloalkyl, C₂-C₁₅-Alkenyl, C₁-C₁₂-Halogenalkyl, C₁-C₁₂-Alkoxy, C₁-C₁₂-Alkylthio, C₆-C₁₀-Aryl, C₆-C₁₀-Aryloxy, C₇-C₁₂-Arylalkyl, -CO₂R₆, -COR₆ oder 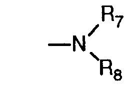 darstellen, wobei mindestens einer der Reste R₁ bis R₅ Wasserstoff, Halogen oder C₁-C₁₅-Alkyl bedeutet, ferner die Reste R₁ und R₂, R₂ und R₃, R₃ und R₄ oder R₄ und R₅ zusammen mit den Kohlenstoffatomen, an die sie gebunden sind, einen Benzo- oder Cyclohexenyl-Ring bilden, und
R₆ C₁-C₂₀-Alkyl, durch Sauerstoff, Schwefel oder 〉N-R₉ unterbrochenes C₂-C₂₀-Alkyl; oder C₇-C₁₂-Arylalkyl bedeutet.

3. Verbindungen gemäss Anspruch 1, worin mindestens zwei der Reste R₁ bis R₅ Wasserstoff sind.

4. Verbindungen gemäss Anspruch 1, worin die Metalle Calcium, Titan, Mangan, Eisen, Kobalt, Zink, Yttrium, Zirkonium, Lanthan oder Cer darstellen.

5. Verbindungen gemäss Anspruch 1, worin das Metall Zirkonium ist.

6. Verbindungen gemäss Anspruch 1, worin
R₁ Wasserstoff bedeutet,
R₂, R₃, R₄ und R₅ unabhängig voneinander Wasserstoff, Chlor, Brom, Nitro, Cyano, CF₃, C₁-C₈-Alkyl, C₅-C₇-Cycloalkyl, C₂-C₈-Alkenyl, C₁-C₈-Alkoxy, C₁-C₈-Alkylthio, Phenyl, Phenyloxy, Benzyl, -CO₂R₆, -COR₆ oder 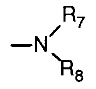 darstellen,
R₆ C₁-C₁₂-Alkyl, durch Sauerstoff unterbrochenes C₂-C₁₂-Alkyl; oder Benzyl bedeutet,
R₇ und R₈ unabhängig voneinander Wasserstoff, C₁-C₈-Alkyl oder durch Sauerstoff unterbrochenes C₂-C₁₂-Alkyl darstellen, und
n eine ganze Zahl aus dem Bereich von 1 bis 5 bedeutet.

7. Verbindungen gemäss Anspruch 1, worin
R₁, R₂ und R₄ Wasserstoff bedeuten,
R₃ und R₅ unabhängig voneinander Wasserstoff, Chlor, Brom, CF₃, C₁-C₈-Alkyl, Cyclohexyl, C₁-C₈-Alkoxy, -CO₂R₆, -COR₆ oder 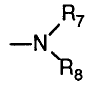 darstellen,
R₆ C₁-C₈-Alkyl bedeutet,
R₇ und R₈ unabhängig voneinander Wasserstoff oder C₁-C₈-Alkyl darstellen, und
n eine ganze Zahl aus dem Bereich von 2 bis 4 bedeutet.

8. Verbindungen gemäss Anspruch 1, worin R₁, R₂, R₄ und R₅ Wasserstoff bedeuten und n 2 ist.

9. Verbindungen gemäss Anspruch 1, worin R₁, R₂, R₄ und R₅ Wasserstoff sind, R₃ Wasserstoff, C₁-C₄-Alkyl oder Chlor bedeutet, n 2 ist und die Metalle Calcium, Titan, Mangan, Eisen, Kobalt, Zink, Yttrium, Zirkonium, Lanthan oder Cer darstellen.

10. Ueberzugszusammensetzung enthaltend
a) ein organisches filmbildendes Bindemittel und
b) als Korrosionsinhibitor mindestens ein Erdalkalimetallsalz, Uebergangsmetallsalz oder Uebergangsmetallkomplex von einer Verbindung der Formel I 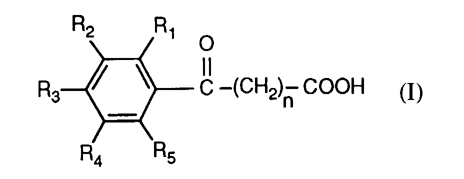
worin R₁, R₂, R₃, R₄ und R₅ unabhängig voneinander Wasserstoff, Halogen, Nitro, Cyano, CF₃, C₁-C₁₅-Alkyl, C₅-C₁₂-Cycloalkyl, C₂-C₁₅-Alkenyl, C₁-C₁₂-Halogenalkyl, C₁-C₁₂-Alkoxy, C₁-C₁₂-Alkylthio, unsubstituiertes oder durch C₁-C₄-Alkyl substituiertes C₆-C₁₀-Aryl; unsubstituiertes oder durch C₁-C₄-Alkyl substituiertes C₆-C₁₀-Aryloxy; unsubstituiertes oder am Arylrest durch 1 bis 3 C₁-C₄-Alkyl substituiertes C₇-C₁₂-Arylalkyl;
-CO₂R₆, -COR₆ oder 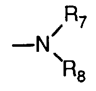 darstellen, wobei mindestens einer der Reste R₁ bis R₅ Wasserstoff, Halogen oder C₁-C₁₅-Alkyl bedeutet, ferner die Reste R₁ und R₂, R₂ und R₃, R₃ und R₄ oder R₄ und R₅ zusammen mit den Kohlenstoffatomen, an die sie gebunden sind, einen Benzo- oder Cyclohexenyl-Ring bilden,
R₆ C₁-C₂₀-Alkyl, durch Sauerstoff, Schwefel oder 〉N-R₉ unterbrochenes C₂-C₂₀-Alkyl; unsubstituiertes oder am Arylrest durch 1 bis 3 C₁-C₄-Alkyl substituiertes C₇-C₁₂-Arylalkyl bedeutet,
R₇ und R₈ unabhängig voneinander Wasserstoff, C₁-C₂₄-Alkyl oder durch Sauerstoff, Schwefel oder 〉N-R₉ unterbrochenes C₂-C₂₄-Alkyl darstellen,
R₉ Wasserstoff oder C₁-C₈-Alkyl bedeutet, und
n eine ganze Zahl aus dem Bereich von 1 bis 10 darstellt.

11. Ueberzugszusammensetzung gemäss Anspruch 10, worin die Ueberzugszusammensetzung ein Anstrichmittel ist.

12. Ueberzugszusammensetzung gemäss Anspruch 10, worin die Ueberzugszusammensetzung ein wässriges Anstrichmittel ist.

13. Ueberzugszusammensetzung gemäss Anspruch 10, worin die Komponente a) ein Epoxidharz, ein Polyurethanharz, ein Polyesterharz, ein Acrylharz, ein Acrylcopolymerharz, ein Polyvinylharz, ein Phenolharz, ein Alkydharz oder eine Mischung solcher Harze ist.

14. Ueberzugszusammensetzung gemäss Anspruch 10, enthaltend zusätzlich eine oder mehrere Komponenten aus der Gruppe der Pigmente, Farbstoffe, Füllstoffe, Fliesskontrollmittel, Dispergiermittel, Thixotropiemittel, Haftungsverbesserer, Antioxidantien, Lichtstabilisatoren oder Härtungskatalysatoren.

15. Ueberzugszusammensetzung gemäss Anspruch 10, worin die Komponente b) in einer Menge von 0,01 bis 20 % bezogen auf das Gesamtgewicht der Ueberzugszusammensetzung vorliegt.

16. Verwendung der Erdalkalimetallsalze, Uebergangsmetallsalze oder Uebergangsmetallkomplexe von Verbindungen der Formel I der in Anspruch 10 definierten Formel I als Korrosionsinhibitoren in Ueberzugszusammensetzungen für metallische Oberflächen.

17. Verfahren zum Schutz eines korrodierbaren Metallsubstrats, dadurch gekennzeichnet, dass man auf dieses eine Ueberzugszusammensetzung gemäss Anspruch 10 aufbringt und sie anschliessend trocknet und/oder härtet.

18. Verfahren zur Herstellung von Erdalkalimetallsalzen, Uebergangsmetallsalzen und Uebergangsmetallkomplexen von Verbindungen der Formel I gemäss Anspruch 1, dadurch gekennzeichnet, dass eine Ketocarbonsäure der Formel I oder ein Alkalimetallsalz davon mit einer Erdalkalimetall- oder Uebergangsmetall-Verbindung umgesetzt wird.

19. Verfahren gemäss Anspruch 18, dadurch gekennzeichnet, dass als Erdalkalimetall- oder Uebergangsmetall-Verbindung ein Salz, eine metallorganische Verbindung oder eine anorganische Metallbase eingesetzt wird.

20. Verfahren gemäss Anspruch 18, dadurch gekennzeichnet, dass das molare Verhältnis der Ketocarbonsäure der Formel I zur Uebergangsmetall-Verbindung 20 : 1 bis 1 : 10 beträgt.

21. Verfahren gemäss Anspruch 18, dadurch gekennzeichnet, dass die Uebergangsmetall-Verbindung eine Zirkonium- oder Titan-Verbindung ist.

## Claims

1. An alkaline earth metal salt, transition metal salt or transition metal complex of a compound of the formula I 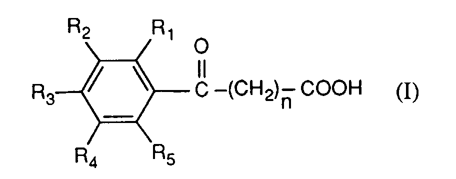 in which R₁, R₂, R₃, R₄ and R₅ are, independently of one another, hydrogen, halogen, nitro, cyano, CF₃, C₁-C₁₅alkyl, C₅-C₁₂cycloalkyl, C₂-C₁₅alkenyl, C₁-C₁₂haloalkyl, C₁-C₁₂alkoxy, C₁-C₁₂alkylthio, C₆-C₁₀aryl which is unsubstituted or substituted by C₁-C₄alkyl; C₆-C₁₀aryloxy which is unsubstituted or substituted by C₁-C₄alkyl;
C₇-C₁₂arylalkyl which is unsubstituted or substituted on the aryl radical by from 1 to 3 C₁-C₄alkyl groups; -CO₂R₆, -COR₆ or 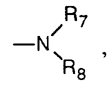 where at least one of the radicals R₁ to R₅ is hydrogen, halogen or C₁-C₁₅alkyl, and also the radicals R₁ and R₂, R₂ and R₃, R₃ and R₄ or R₄ and R₅ form, together with the carbon atoms to which they are bound, a benzo or cyclohexenyl ring,
R₆ is C₁-C₂₀alkyl, C₂-C₂₀alkyl interrupted by oxygen, sulfur or 〉N-R₉ ; C₇-C₁₂arylalkyl which is unsubstituted or substituted on the aryl radical by from 1 to 3 C₁-C₄alkyl groups,
R₇ and R₈ are, independently of one another, hydrogen, C₁-C₂₄alkyl or C₂-C₂₄alkyl which is interrupted by oxygen, sulfur or 〉N-R₉ ,
R₉ is hydrogen or C₁-C₈alkyl, and
n is an integer in the range from 1 to 10; with the proviso that the calcium salt of 3-(3-chloro-4-isopropylbenzoyl)propionic acid, the calcium salt of 3-(3-chloro-4-cyclohexylbenzoyl)propionic acid and the calcium, magnesium and zinc salt of 4-benzoylbutyric acid are excluded.

2. A compound according to claim 1, in which
R₁, R₂, R₃, R₄ and R₅ are, independently of one another, hydrogen, halogen, nitro, cyano, CF₃, C₁-C₁₅alkyl, C₅-C₁₂cycloalkyl, C₂-C₁₅alkenyl, C₁-C₁₂haloalkyl, C₁-C₁₂alkoxy, C₁-C₁₂alkylthio, C₆-C₁₀aryl, C₆-C₁₀aryloxy, C₇-C₁₂arylalkyl, -CO₂R₆, -COR₆ or 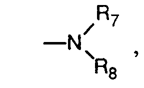 where at least one of the radicals R₁ to R₅ is hydrogen, halogen or C₁-C₁₅alkyl, and also the radicals R₁ and R₂, R₂ and R₃, R₃ and R₄ or R₄ and R₅ form, together with the carbon atoms to which they are bound, a benzo or cyclohexenyl ring, and
R₆ is C₁-C₂₀alkyl, C₂-C₂₀alkyl interrupted by oxygen, sulfur or 〉N-R₉ ; or C₇-C₁₂arylalkyl.

3. A compound according to claim 1, in which at least two of the radicals R₁ to R₅ are hydrogen.

4. A compound according to claim 1, in which the metal is calcium, titanium, manganese, iron, cobalt, zinc, yttrium, zirconium, lanthanum or cerium.

5. A compound according to claim 1, in which the metal is zirconium.

6. A compound according to claim 1, in which
R₁ is hydrogen,
R₂, R₃, R₄ and R₅ are, independently of one another, hydrogen, chlorine, bromine, nitro, cyano, CF₃, C₁-C₈alkyl, C₅-C₇cycloalkyl, C₂-C₈alkenyl, C₁-C₈alkoxy, C₁-C₈alkylthio, phenyl, phenyloxy, benzyl, -CO₂R₆, -COR₆ or 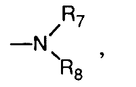 R₆ is C₁-C₁₂alkyl, C₂-C₁₂alkyl interrupted by oxygen; or benzyl,
R₇ and R₈ are, independently of one another, hydrogen, C₁-C₈alkyl or C₂-C₁₂alkyl interrupted by oxygen, and
n is an integer in the range from 1 to 5.

7. A compound according to claim 1, in which
R₁, R₂ and R₄ are hydrogen,
R₃ and R₅ are, independently of one another, hydrogen, chlorine, bromine, CF₃, C₁-C₈alkyl, cyclohexyl, C₁-C₈alkoxy, -CO₂R₆, -COR₆ or 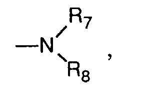 R₆ is C₁-C₈alkyl,
R₇ and R₈ are, independently of one another, hydrogen or C₁-C₈alkyl, and
n is an integer in the range from 2 to 4.

8. A compound according to claim 1, in which R₁, R₂, R₄ and R₅ are hydrogen and n is 2.

9. A compound according to claim 1, in which R₁, R₂, R₄ and R₅ are hydrogen, R₃ is hydrogen, C₁-C₄alkyl or chlorine, n is 2 and the metal is calcium, titanium, manganese, iron, cobalt, zinc, yttrium, zirconium, lanthanum or cerium.

10. A coating composition comprising
a) an organic film-forming binder and
b) as corrosion inhibitor at least one alkaline earth metal salt, transition metal salt or transition metal complex of a compound of the formula I 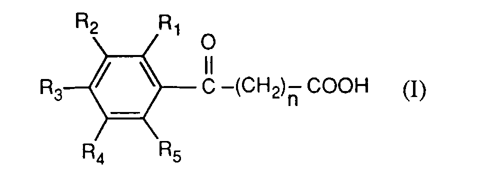
in which R₁, R₂, R₃, R₄ and R₅ are, independently of one another, hydrogen, halogen, nitro, cyano, CF₃, C₁-C₁₅alkyl, C₅-C₁₂cycloalkyl, C₂-C₁₅alkenyl, C₁-C₁₂haloalkyl, C₁-C₁₂alkoxy, C₁-C₁₂alkylthio, C₆-C₁₀aryl which is unsubstituted or substituted by C₁-C₄alkyl; C₆-C₁₀aryloxy which is unsubstituted or substituted by C₁-C₄alkyl;
C₇-C₁₂arylalkyl which is unsubstituted or substituted on the aryl radical by from 1 to 3 C₁-C₄alkyl groups; -CO₂R₆, -COR₆ or 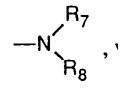 where at least one of the radicals R₁ to R₅ is hydrogen, halogen or C₁-C₁₅alkyl, and also the radicals R₁ and R₂, R₂ and R₃, R₃ and R₄ or R₄ and R₅ form, together with the carbon atoms to which they are bound, a benzo or cyclohexenyl ring,
R₆ is C₁-C₂₀alkyl, C₂-C₂₀alkyl interrupted by oxygen, sulfur or 〉N-R₉ ; C₇-C₁₂arylalkyl which is unsubstituted or substituted on the aryl radical by from 1 to 3 C₁-C₄alkyl groups,
R₇ and R₈ are, independently of one another, hydrogen, C₁-C₂₄alkyl or C₂-C₂₄alkyl which is interrupted by oxygen, sulfur or 〉N-R₉ ,
R₉ is hydrogen or C₁-C₈alkyl, and
n is an integer in the range from 1 to 10.

11. A coating composition according to claim 10, wherein the coating composition is a liquid.

12. A coating composition according to claim 10, wherein the coating composition is a water-based liquid.

13. A coating composition according to claim 10, wherein the component a) is an epoxy resin, a polyurethane resin, a polyester resin, an acrylic resin, an acrylic copolymer resin, a polyvinyl resin, a phenolic resin, an alkyd resin or a mixture of such resins.

14. A coating composition according to claim 10, additionally comprising one or more components from the group consisting of pigments, dyes, fillers, flow modifiers, dispersants, thixotropes, adhesion promoters, antioxidants, light stabilizers and curing catalysts.

15. A coating composition according to claim 10, wherein the component b) is present in an amount from 0.01 to 20 % based on the total weight of the coating composition.

16. Use of an alkaline earth metal salt, transition metal salt or transition metal complex of a compound of the formula I defined in claim 10 as corrosion inhibitor in coating compositions for metal surfaces.

17. A process for protecting a corrodable metal substrate, which comprises applying to this a coating composition according to claim 10 and subsequently drying and/or curing the coating.

18. A process for preparing alkaline earth metal salts, transition metal salts and transition metal complexes of compounds of the formula I according to claim 1, which comprises reacting a ketocarboxylic acid of the formula I or an alkali metal salt thereof with an alkaline earth metal compound or transition metal compound.

19. A process according to claim 18, wherein the alkaline earth metal compound or transition metal compound used is a salt, an organometallic compound or an inorganic metal base.

20. A process according to claim 18, wherein the molar ratio of the ketocarboxylic acid of the formula I to the transition metal compound is from 20:1 to 1:10.

21. A process according to claim 18, wherein the transition metal compound is a zirconium or titanium compound.

## Revendications

1. Sels de métaux alcalino-terreux, sels de métaux de transition et complexes de métaux de transition de composés de formule I 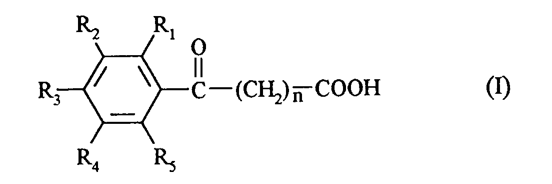 dans laquelle
R₁, R₂, R₃, R₄ et R₅ représentent indépendamment les uns des autres un atome d'hydrogène ou d'halogène ou un groupe nitro, cyano, CF₃, alkyle en C₁-C₁₅, cycloalkyle en C₅-C₁₂, alcényle en C₂-C₁₅, halogénoalkyle en C₁-C₁₂, alcoxy en C₁-C₁₂, alkylthio en C₁-C₁₂ aryle en C₆-C₁₀ non substitué ou substitué par alkyle en C₁-C₄; aryloxy en C₆-C₁₀ non substitué ou substitué par alkyle en C₁-C₄; arylalkyle en C₇-C₁₂ non substitué ou substitué sur le reste aryle par 1 à 3 restes alkyle en C₁-C₄; -CO₂R₆, -COR₆ ou 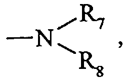 au moins l'un des restes R₁ à R₅ étant un atome d'hydrogène ou d'halogène ou un reste alkyle en C₁-C₁₅, et les restes R₁ et R₂, R₂ et R₃, R₃ et R₄ ou R₄ et R₅ ensemble peuvent aussi former avec les atomes de carbone auxquels ils sont liés un cycle benzo ou cyclohexényle,
R₆ représente un reste alkyle en C₁-C₂₀, alkyle en C₂-C₂₀ interrompu par de l'oxygène, du soufre ou 〉N-R₉ ; arylalkyle en C₇-C₁₂ non substitué ou substitué sur le reste aryle par 1 à 3 restes alkyle en C₁-C₄;
R₇ et R₈ représentent indépendamment l'un de l'autre un atome d'hydrogène ou un reste alkyle en C₁-C₂₄ ou alkyle en C₂-C₂₄ interrompu par de l'oxygène, du soufre ou 〉N-R₉ ;
R₉ représente un atome d'hydrogène ou un reste alkyle en C₁-C₈, et
n est un nombre entier compris entre 1 et 10; à condition que soient exclus le sel de calcium de l'acide 3-(3-chloro-4-isopropylbenzoyl)propionique, le sel de calcium de l'acide 3-(3-chloro-4-cyclohexylbenzoyl)propionique et les sels de calcium, de magnésium et de zinc de l'acide 4-benzoylbutyrique.

2. Composés selon la revendication 1, dans lesquels
R₁, R₂, R₃, R₄ et R₅ représentent indépendamment les uns des autres un atome d'hydrogène ou d'halogène ou un groupe nitro, cyano, CF₃, alkyle en C₁-C₁₅, cycloalkyle en C₅-C₁₂, alcényle en C₂-C₁₅, halogénoalkyle en C₁-C₁₂, alcoxy en C₁-C₁₂, alkylthio en C₁-C₁₂, aryle en C₆-C₁₀, aryloxy en C₆-C₁₀, arylalkyle en C₇-C₁₂, -CO₂R₆, -COR₆ ou 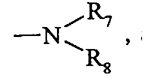 au moins l'un des restes R₁ à R₅ étant un atome d'hydrogène ou d'halogène ou un reste alkyle en C₁-C₁₅, et les restes R₁ et R₂, R₂ et R₃, R₃ et R₄ ou R₄ et R₅ ensemble peuvent aussi former avec les atomes de carbone auxquels ils sont liés un cycle benzo ou cyclohexényle, et
R₆ représente un reste alkyle en C₁-C₂₀, alkyle en C₂-C₂₀ interrompu par de l'oxygène, du soufre ou 〉N-R₉ ; ou arylalkyle en C₇-C_{C2}.

3. Composés selon la revendication 1, dans lesquels au moins deux des restes R₁ à R₅ sont des atomes d'hydrogène.

4. Composés selon la revendication 1, dans lesquels les métaux sont le calcium, le titane, le manganèse, le fer, le cobalt, le zinc, l'yttrium, le zirconium, le lanthane ou le cérium.

5. Composés de formule I selon la revendication 1, dans lesquels le métal est le zirconium.

6. Composés selon la revendication 1, dans lesquels
R₁ représente un atome d'hydrogène,
R₂, R₃, R₄ et R₅ sont indépendamment les uns des autres un atome d'hydrogène, de chlore ou de brome ou un reste nitro, cyano, CF₃, alkyle en C₁-C₈, cycloalkyle en C₅-C₇, alcényle en C₂-C₈, alcoxy en C₁-C₈, alkylthio en C₁-C₈, phényle, phényloxy, benzyle, -CO₂R₆, -COR₆ ou 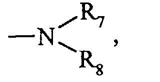 R₆ représente un reste alkyle en C₁-C₁₂; alkyle en C₂-C₁₂ interrompu par de l'oxygène; ou benzyle,
R₇ et R₈ représentent indépendamment l'un de l'autre un atome d'hydrogène ou un reste alkyle en C₁-C₈ ou alkyle en C₂-C₁₂ interrompu par de l'oxygène, et
n est un nombre entier compris entre 1 et 5.

7. Composés selon la revendication 1, dans lesquels
R₁, R₂ et R₄ représentent des atomes d'hydrogène,
R₃ et R₅ représentent indépendamment l'un de l'autre un atome d'hydrogène, de chlore ou de brome ou un reste CF₃, alkyle en C₁-C₈, cyclohexyle, alcoxy en C₁-C₈, -CO₂R₆, -COR₆ ou 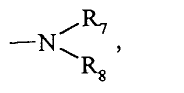 R₆ représente un reste alkyle en en C₁-C₈,
R₇ et R₈ représentent indépendamment l'un de l'autre un atome d'hydrogène ou un reste alkyle en C₁-C₈ et
n est un nombre entier compris entre 2 et 4.

8. Composés selon la revendication 1, dans lesquels R₁, R₂, R₄ et R₅ sont des atomes d'hydrogène et n est 2.

9. Composés selon la revendication 1, dans lesquels R₁, R₂, R₄ et R₅ sont des atomes d'hydrogène, R₃ est un atome d'hydrogène, un reste alkyle en C₁-C₄ ou un atome de chlore, n est 2 et les métaux sont le calcium, le titane, le manganèse, le fer, le cobalt, le zinc, l'yttrium, le zirconium, le lanthane ou le cérium.

10. Composition de revêtement contenant
a) un liant filmogène organique et
b) comme inhibiteur de corrosion, au moins un sel de métal alcalino-terreux, un sel de métal de transition ou un complexe de métal de transition d'un composé de formule I 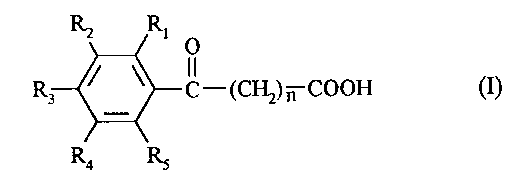
dans laquelle
R₁, R₂, R₃, R₄ et R₅ représentent indépendamment les uns des autres un atome d'hydrogène ou d'halogène ou un groupe nitro, cyano, CF₃, alkyle en C₁-C₁₅, cycloalkyle en C₅-C₁₂, alcényle en C₂-C₁₅, halogénoalkyle en C₁-C₁₂, alcoxy en C₁-C₁₂, alkylthio en C₁-C₁₂, aryle en C₆-C₁₀ non substitué ou substitué par alkyle en C₁-C₄; aryloxy en C₆-C₁₀ non substitué ou substitué par alkyle en C₁-C₄; arylalkyle en C₇-C₁₂ non substitué ou substitué sur le reste aryle par 1 à 3 restes alkyle en C₁-C₄; -CO₂R₆, -COR₆ ou 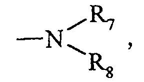 au moins l'un des restes R₁ à R₅ étant un atome d'hydrogène ou d'halogène ou un reste alkyle en C₁-C₁₅, et les restes R₁ et R₂, R₂ et R₃, R₃ et R₄ ou R₄ et R₅ ensemble peuvent aussi former avec les atomes de carbone auxquels ils sont liés un cycle benzo ou cyclohexényle,
R₆ représente un reste alkyle en C₁-C₂₀, alkyle en C₂-C₂₀ interrompu par de l'oxygène, du soufre ou 〉N-R₉ ; arylalkyle en C₇-C₁₂ non substitué ou substitué sur le reste aryle par 1 à 3 restes alkyle en C₁-C₄;
R₇ et R₈ représentent indépendamment l'un de l'autre un atome d'hydrogène ou un reste alkyle en C₁-C₂₄ ou alkyle en C₂-C₂₄ interrompu par de l'oxygène, du soufre ou 〉N-R₉ ;
R₉ représente un atome d'hydrogène ou un reste alkyle en C₁-C₈, et
n est un nombre entier compris entre 1 et 10.

11. Composition de revêtement selon la revendication 10, dans laquelle la composition de revêtement est une composition de peinture.

12. Composition de revêtement selon la revendication 10, dans laquelle la composition de revêtement est une composition de peinture à l'eau.

13. Composition de revêtement selon la revendication 10, dans laquelle le constituant a) est une résine époxy, une résine polyuréthane, une résine polyester, une résine acrylique, un résine copolymère acrylique, une résine polyvinylique, une résine phénolique, une résine alkyde ou un mélange de telles résines.

14. Composition de revêtement selon la revendication 10, contenant en outre un ou plusieurs constituants du groupe des pigments, des colorants, des charges, des agents de réglage de la fluidité, des agents dispersants, des agents thixotropes, des agents adhésifs, des antioxydants, des stabilisants à la lumière ou des catalyseurs de durcissement.

15. Composition de revêtement selon la revendication 10, dans laquelle le constituant b) se trouve en une quantité de 0,01 à 20 % par rapport à la masse totale de la composition de revêtement.

16. Utilisation des sels de métaux alcalino-terreux, des sels de métaux de transition et des complexes de métaux de transition de composés de formule I ayant la formule I définie dans la revendication 10 comme inhibiteurs de corrosion dans des compositions de revêtement pour des surfaces métalliques.

17. Procédé de protection d'un substrat métallique attaquable par corrosion, caractérisé en ce que l'on applique sur ce substrat une composition de revêtement selon la revendication 10, puis on la sèche et/ou on la durcit.

18. Procédé de préparation de sels de métaux alcalino-terreux, de sels de métaux de transition et de complexes de métaux de transition de composés de formule I selon la revendication 1, caractérisé en ce que l'on fait réagir un acide cétocarboxylique de formule I ou un de ses sels de métaux alcalins avec un composé de métal alcalino-terreux ou de métal de transition.

19. Procédé selon la revendication 18, caractérisé en ce que le composé de métal alcalino-terreux ou de métal de transition est un sel, un composé organométallique ou une base métallique inorganique.

20. Procédé selon la revendication 18, caractérisé en ce que le rapport molaire de l'acide cétocarboxylique de formule I au composé de métal de transition est compris entre 20:1 et 1:10.

21. Procédé selon la revendication 18, caractérisé en ce que le composé de métal de transition est un composé de zirconium ou de titane.
